# EUROPÄISCHE PATENTSCHRIFT

(11) **EP 2 023 888 B1**
(45) Veröffentlichungstag und Bekanntmachung des Hinweises auf die Patenterteilung: **07.10.2015**
(21) Anmeldenummer: 07724504.1
(22) Anmeldetag: 24.04.2007
(51) Int. Cl.: A61K 8/34, A61K 8/39, A61K 8/73, A61K 8/86, A61Q 15/00, A61K 8/06, A61Q 19/00

(54) **SCHNELL TROCKNENDE KOSMETISCHE EMULSIONEN ZUR ROLL-ON-APPLIKATION**
QUICK-DRYING COSMETIC EMULSIONS FOR ROLL-ON APPLICATION
ÉMULSIONS COSMÉTIQUES À SÉCHAGE RAPIDE

(30) Priorität: 28.04.2006 DE 102006020382
(43) Veröffentlichungstag der Anmeldung: 18.02.2009
(73) Patentinhaber: Henkel AG & Co. KGaA, 40589 Düsseldorf (DE)
(72) Erfinder: TECKENBROCK, Gertraud, 45549 Sprockhövel (DE); HEIDE, Barbara, 47809 Krefeld (DE)
(86) Internationale Anmeldenummer: PCT/EP2007/003574
(87) Internationale Veröffentlichungsnummer: WO 2007/124889

(56) Entgegenhaltungen:
- EP-A- 1 584 370
- EP-A1- 0 265 087
- EP-A2- 0 659 404
- WO-A-00/21498
- DE-A1- 10 237 460
- GB-A- 2 013 085
- US-A- 5 356 612
- US-A1- 2005 238 598

## Beschreibung

Die vorliegende Anmeldung betrifft kosmetische Öl-in-Wasser-Emulsionen, die insbesondere zur Applikation mit einem Rollapplikator geeignet sind und eine hohe Lagerstabilität, ein nicht-fettendes Hautgefühl und eine besonders schnelle Trocknungscharakteristik aufweisen, wobei die Emulsionen einen geringen Gehalt an Öl- oder Fettphase sowie mindestens ein Polysaccharid enthalten.

Es gibt zahlreiche Möglichkeiten, kosmetische Zusammensetzungen zur Haut- und Körperpflege auf die Haut aufzutragen. Cremes, Salben und Lotionen werden üblicherweise aus einem Tiegel, einer Tube oder einem Pumpspender entnommen und mit der Hand aufgetragen und verrieben. Formstabile Stiftmassen werden aus einem Stiftspender heraus über die Haut gestrichen, bis eine wirksame Menge aufgetragen ist. Auch Gele und Cremes können mit stiftähnlichen Dispensern, die mit einer Dispenseroberfläche über die Haut gestrichen werden, aufgetragen werden. Insbesondere für schweißhemmende und/oder desodorierende Zusammensetzungen für den Unterarmbereich wurden zahlreiche verschiedene Applikationsformen entwickelt, neben den bereits genannten vor allem die treibgashaltigen und treibgasfreien Sprays und die Roll-on-Zusammensetzungen. Bei letzteren wird eine leicht verdickte Flüssigkeit aus einem Vorratsbehälter über eine drehbar gelagerte Kugel durch Rollen über die Haut appliziert. Roll-on-Applikatoren werden zwar hauptsächlich für den Unterarmbereich eingesetzt, sind aber prinzipiell auch für die Pflege der Gesichtshaut und des Körpers geeignet. Für die Gesichtspflege dienen klein-dimensionierte Roll-on-Applikatoren insbesondere zum Auftragen höher konzentrierter Wirkstoff-Seren auf ausgewählte Problemzonen, z.B. Antifalten-Produkte für die Augenwinkel, die Stirn oder die Oberlippenregion, Anti-Akne-Produkte und Anti-Pickel-Produkte. Dies sorgt für einen (auch ökonomisch) effizienten Einsatz der wertvollen Wirkstoffe. Außerdem kann so der Einsatz von höher konzentrierten Wirkstoffen, die großflächig ein unangenehmes Hautgefühl erzeugen könnten (z.B. der Anti-Pickel-Wirkstoff Salicylsäure), lokal beschränkt werden. Gleichzeitig erlaubt der Applikator ein bequemes, zeitsparendes Auftragen. Zahlreiche kosmetische Wirkstoff sind wasserlöslich und ihre Freisetzung auf der Haut könnte durch Öl- und Fettbestandteile des Kosmetikums verzögert werden. Als rein wässrige Lösung wäre das Produkt allerdings kaum zu dosieren und damit für den Verbraucher nicht akzeptabel. Durch leichtes Andicken jedoch lässt sich eine solche Zusammensetzung bequem mit einem Roll-on-Applikator verwenden. Häufig werden polymere Verdickungsmittel eingesetzt. Nachteilig hierbei ist, dass die meisten polymeren Verdickungsmittel in den erforderlichen Konzentrationen ein sehr klebriges Hautgefühl erzeugen. Darüber hinaus weisen viele dieser Verdicker keine zusätzlichen kosmetischen Pflegeeffekte auf. Eine vorteilhafte Alternative hierzu stellen Emulsionen mit einem geringen Öl- und Fettgehalt dar. Die Emulsionsbildung führt auch ohne Polymerverdicker zu einem Anstieg der Viskosität. Der Öl- und Fettanteil der Emulsion entfaltet darüber hinaus eine hautpflegende Wirkung.
Emulsionen sind, im Gegensatz zu Mikroemulsionen, thermodynamisch instabil. Die thermodynamisch stabilen Mikroemulsionen können meist nur durch einen relativ hohen Emulgatorgehalt stabilisiert werden. Ein hoher Gehalt an Emulgatoren kann jedoch im ungünstigsten Fall hautreizend wirken und wird daher möglichst vermieden. Außerdem bilden sich Mikroemulsionen häufig nur in einem sehr engen Mischungsbereich der einzelnen Komponenten. Bei kosmetischen Zusammensetzungen mit mehreren Bestandteilen kann es daher entwicklungstechnisch mitunter sehr schwierig sein, geeignete Mikroemulsionsbereiche einzustellen. Emulsionen sind für eine gewisse Zeit stabil, weil die Koaleszenz der dispergierten Tröpfchen kinetisch gehemmt ist. Diese kinetische Hemmung kann aufgehoben werden durch Lagerung bei hohen Temperaturen (relevant insbesondere für die Produktion und Vermarktung in warmen Ländern) oder bei Lagerung unter größeren Temperaturschwankungen (z. B. in unzureichend klimatisierten Verkaufsräumen, beim Transport über weitere Strecken). Auch die hohe Salzkonzentration in Antitranspirant-Zusammensetzungen, bedingt durch die relativ hohe Konzentration an schweißhemmenden Wirkstoffen, kann die Emulsionsdestabilisierung begünstigen (z. B. durch Aussalzeffekte).
Bei Roll-on-Emulsionen mit einem typischerweise hohen Wasseranteil kann das feuchte Hautgefühl direkt nach der Applikation vom Verbraucher als unangenehm wahrgenommen werden.

Aus EP 270328 A2 sind schweißhemmende Öl-in-Wasser-Emulsionen bekannt, die mit einem hohen Gehalt an Polysacchariden eine Verkapselung der enthaltenen Parfümöle erzielen. Der hohe Polysaccharidgehalt kann sich aber ungünstig vor allem auf das Hautgefühl auswirken. Außerdem kann der hohe Polysaccharidgehalt auch die Lagerstabilität der Emulsionen, insbesondere bei höheren Lagertemperaturen von 45 °C und darüber, beeinträchtigen.
Aus US 4499069 sind schweißhemmende Öl-in-Wasser-Emulsionen bekannt, die etwa 22 Gew.-% einer Ölphase, umfassend flüchtige Siliconöle und PPG-15-Stearylether, Steareth-2, Steareth-21 sowie 2 Gew.-% Aluminiumstärkeoctenylsuccinat enthalten. Diese Emulsionen sind zwar als lagerstabil bezeichnet, allerdings ist auch angegeben, dass sie nach 4 Wochen Lagerung bei 45 °C ein Aufrahmen der dispergierten Phase zeigen. Dieses Stabilitätsverhalten ist für heutige Verbraucheransprüche nicht mehr ausreichend.
Aus US 6261543 sind schweißhemmende Öl-in-Wasser-Emulsionen bekannt, die etwa 6,5 - 10 Gew.-% einer Öl- und/oder Fettphase, ein Gemisch aus hydrophilen und lipophilen Emulgatoren sowie 1 Gew.-% einer amphoteren oder kationischen Stärke enthalten. Ein entsprechendes Vergleichsbeispiel mit einer nichtionischen Stärke wurde als bei 50 °C nicht lagerstabil angegeben. EP 1584370 A1 offenbart eine schweißhemmende Lotion mit einer Viskosität von 2050 mPas, die 4 Gew.-% PPG-15 Stearylether, 4 Gew.-% eines Stärkederivats, einen Öl-in-Wasser-Emulgator, einen Wasser-in-Öl-Emulgator und ca. 69 Gew.-% Wasser enthält.
US 5356612 beschäftigt sich mit der Herstellung eines basischen Aluminiumchlorhydroxids mit verbesserter schweißhemmender Wirkung, das mit Monokieselsäure modifiziert wurde, und offenbart eine schweißhemmende wässrige Roll-on-Zusammensetzung, die 2 Gew.-% Aluminiumstärkeoctenylsuccinat und 21,8 Gew.% Ölphase (Cyclomethicone, PPG-15 Stearylether) enthält. Keines dieser Dokumente beschäftigt sich mit dem Problem des beschleunigten Trocknens der Roll-on-Emulsion auf der Haut.

Eine Aufgabe der vorliegenden Erfindung war es, schweißhemmende Öl-in-Wasser-Emulsionen mit verbesserter Lagerstabilität, insbesondere verlängerter Lagerstabilität bei Temperaturen von 40 °C und darüber, bereitzustellen. Eine weitere Aufgabe der vorliegenden Erfindung war es, schweißhemmende Öl-in-Wasser-Emulsionen mit einem nicht-fettenden Hautgefühl bereitzustellen. Eine weitere Aufgabe der vorliegenden Erfindung war es, schweißhemmende Öl-in-Wasser-Emulsionen bereitzustellen, die möglichst schnell auf der Haut trocknen.
Eine weitere Aufgabe der vorliegenden Erfindung war es, kosmetische Öl-in-Wasser-Emulsionen mit verbesserter Lagerstabilität, insbesondere verlängerter Lagerstabilität bei Temperaturen von 40 °C und darüber, zur desodorierenden Hautbehandlung bereitzustellen. Eine weitere Aufgabe der vorliegenden Erfindung war es, kosmetische Öl-in-Wasser-Emulsionen mit einem nicht-fettenden Hautgefühl zur desodorierenden Hautbehandlung bereitzustellen. Eine weitere Aufgabe der vorliegenden Erfindung war es, kosmetische Öl-in-Wasser-Emulsionen, die möglichst schnell auf der Haut trocknen, zur desodorierenden Hautbehandlung bereitzustellen.
Überraschend wurde gefunden, dass Öl-in-Wasser-Emulsionen mit einem Anteil an Öl- oder Fettphase von maximal 6,5 Gew.-%, enthaltend ausgewählte Ölkomponenten in Kombination mit einem, vorzugsweise geringen, Anteil an mindestens einem Polysaccharid, hergestellt werden können, die auch bei hohen Temperaturen von 45 °C und darüber mehrere Wochen lang lagerstabil sind und die darüber hinaus nach dem Auftrag auf die Haut eine Trockengeschwindigkeit aufweisen, die vom Anwender als gegenüber dem Stand der Technik deutlich verkürzt wahrgenommen wird.
Ein erster Gegenstand der vorliegenden Erfindung ist daher eine kosmetische Öl-in-Wasser-Emulsion, die keine Mikroemulsion darstellt, enthaltend 0,5 - 6,5 Gew.-% Öl- oder Fettphase, umfassend mindestens eine bei 20 °C flüssige Ölkomponente, ausgewählt aus linearen und verzweigten gesättigten ein- oder mehrwertigen C₃ - C₃₀-Alkanolen, die mit mindestens einer Propylenoxid-Einheit pro Molekül verethert sind, Propylenglycolmonoestern von verzweigten gesättigten C₆ - C₃₀-Alkancarbonsäuren und verzweigten gesättigten C₁₀ - C₃₀-Alkanolen, mindestens 60 Gew.-% Wasser, 0,00001 - 38 Gew.-% mindestens eines kosmetischen Wirkstoffes, ausgewählt aus
- schweißhemmenden Wirkstoffen,
- desodorierenden Wirkstoffen,
- Monomeren, Oligomeren und Polymeren von Aminosäuren, N-C₂-C₂₄-Acylaminosäuren, den Estern und/oder den physiologisch verträglichen Salzen dieser Substanzen,
- DNA- oder RNA-Oligonucleotiden,
- natürlichen Betainverbindungen,
- Vitaminen, Provitaminen und Vitaminvorstufen der Gruppen A, B, C, E, H und K und den Estern der vorgenannten Substanzen,
- α-Hydroxycarbonsäuren, α-Ketocarbonsäuren, β-Hydroxycarbonsäuren und deren Ester-, Lacton- oder Salzform,
- Flavonoiden und Flavonoid-reichen Pflanzenextrakten,
- Isoflavonoiden und Isoflavonoid-reichen Pflanzenextrakten,
- Polyphenolen und Polyphenol-reichen Pflanzenextrakten,
- Ubichinon und Ubichinol sowie deren Derivaten,
- Silymarin,
- Ectoin,
- Repellentien,
- anorganischen und organischen UV-Filtersubstanzen,
- selbstbräunenden Wirkstoffen,
- hautaufhellenden Wirkstoffen,
- hautberuhigenden Wirkstoffen,
- feuchtigkeitsspendenden Wirkstoffen,
- sebumregulierenden Wirkstoffen,
wobei mindestens ein schweißhemmender Wirkstoff enthalten ist,
und mindestens ein Polysaccharid in einer Gesamtmenge von 0,01 - 0,2 Gew.-%, mindestens einen nichtionischen Emulgator mit einem HLB-Wert im Bereich von 3 - 6 in einer Gesamtmenge von 1,8 - 3 Gew.-%, mindestens einen nichtionischen Emulgator mit einem HLB-Wert im Bereich von 12 - 18, ausgewählt aus Steareth, Ceteth, Myristeth, Laureth, Trideceth, Arachideth und Beheneth mit jeweils 7 - 40 Ethylenoxid-Einheiten pro Molekül, in einer Gesamtmenge von 1 - 2 Gew.-%, wobei alle Mengenangaben auf das Gesamtgewicht der Emulsion bezogen sind und das Gewichtsverhältnis von nichtionischen Emulgatoren mit einem HLB-Wert im Bereich von 3 - 6 und nichtionischen Emulgatoren mit einem HLB-Wert im Bereich von 12 - 18 von 0,9 bis 3, bevorzugt 1,3 - 1,9 beträgt und die Emulgatoren definitionsgemäß nicht zur Öl- oder Fettphase zählen, gekennzeichnet durch eine Viskosität im Bereich von 1000 - 5000 mPas, gemessen 1 Tag nach Herstellung mit einem Brookfield-Viskosimeter, Spindel RV 4, 20 s⁻¹, ohne Helipath, bei 20 °C Umgebungstemperatur und 20 °C Probentemperatur.
Die erfindungsgemäßen Öl-in-Wasser-Emulsionen zeichnen sich durch einen Gehalt an mindestens einem Polysaccharid aus. Überraschend wurde festgestellt, dass der Polysaccharid-Gehalt das Trocknen der Emulsion auf der Haut beschleunigt. Gegenüber einer Polysaccharid-freien Emulsion werden erfindungsgemäße Emulsionen von den Testpersonen als auf der Haut schneller trocknend wahrgenommen.
Polysaccharide (Glycane, Polyglycane) ist die Sammelbezeichnung für makromolekulare Kohlenhydrate, deren Moleküle aus einer großen Zahl (mindestens >10, gewöhnlich jedoch erheblich mehr) glycosidisch miteinander verknüpfter Monosaccharid-Moleküle (Glycosen) bestehen.
Zu den erfindungsgemäß bevorzugten Polysacchariden gehören vor allem die Biopolymere Stärke, Cellulose und Dextran, die als Polykondensationsprodukt der D-Glucose aufgefasst werden können (Polyglucosane, Glucane), Inulin als Polykondensat der D-Fructose (Polyfructosan, Fructan), Chitin und Alginsäure.

Unter erfindungsgemäß geeigneten Polysacchariden werden sowohl nicht-modifizierte Polysaccharide, wie beispielsweise Xanthan oder Stärke, als auch chemisch modifizierte Polysaccharid-Derivate, wie beispielsweise Aluminiumstärkeoctenylsuccinat, Hydroxypropylmethylcellulose oder dehydratisiertes Xanthan (INCI: Dehydroxanthan Gum), als auch physikalisch modifizierte Polysaccharide, beispielsweise eine durch thermische Behandlung vorverkleisterte Stärke, verstanden. Erfindungsgemäß bevorzugte Polysaccharide sind ausgewählt aus Stärken, insbesondere aus Mais, Kartoffeln und Weizen, deren Bestandteilen wie Amylose und Amylopektin, Stärkehydrolysaten und Stärkeabbauprodukten, wie Maltodextrin, den physikalisch oder chemisch modifizierten Stärkederivaten, insbesondere den anionischen Stärkederivaten Aluminiumstärkeoctenylsuccinat, Natriumstärkeoctenylsuccinat, Calciumstärkeoctenylsuccinat, Distärkephosphaten, Hydroxyethylstärkephosphaten, Hydroxypropylstärkephosphaten, Natriumcarboxymethylstärken und Natriumstärkeglycolat, Cellulose, den chemisch modifizierten Cellulosederivaten Methylcellulose, Hydroxypropylcellulose, Hydroxyethylcellulose, Hydroxypropylmethylcellulose, Hydroxypropylethylcellulose, Hydroxyethylmethylcellulose und Carboxymethylcellulose. Polysaccharide, die Gums oder Gummen bilden, wie beispielsweise Guar-Gum, Xanthan-Gum, Dehydroxanthan Gum, Alginate, insbesondere Natriumalginat, Gummi arabicum, Karaya-Gummi, Carrageenane, Johannisbrotkernmehl, Leinsamen-Gums und Agar-Agar, können ebenfalls enthalten sein, sind aber weniger bevorzugt. In einer besonders bevorzugten Ausführungsform sind die erfindungsgemäßen Zusammensetzungen frei von Polysaccharid-Gums. In einer weiteren besonders bevorzugten Ausführungsform sind die erfindungsgemäßen Zusammensetzungen frei von Guar-Gum, Xanthan-Gum, Dehydroxanthan Gum, Alginate, insbesondere Natriumalginat, Gummi arabicum, Karaya-Gummi, Carrageenane, Johannisbrotkernmehl, Leinsamen-Gums und Agar-Agar.

Besonders bevorzugte erfindungsgemäße Öl-in-Wasser-Emulsionen sind dadurch gekennzeichnet, dass das mindestens eine Polysaccharid ausgewählt ist aus anionischen und nichtionischen Polysacchariden sowie Mischungen hiervon.

Weitere besonders bevorzugte erfindungsgemäße Öl-in-Wasser-Emulsionen sind dadurch gekennzeichnet, dass das mindestens eine Polysaccharid ausgewählt ist aus anionischen und nichtionischen Polysacchariden, die keine Polysaccharid-Gums bilden.

Weitere besonders bevorzugte erfindungsgemäße Öl-in-Wasser-Emulsionen sind dadurch gekennzeichnet, dass das anionische Polysaccharid ausgewählt ist aus Aluminiumstärkeoctenylsuccinat, Natriumstärkeoctenylsuccinat, Calciumstärkeoctenylsuccinat, Distärkephosphaten, Hydroxyethylstärkephosphaten, Hydroxypropylstärkephosphaten, Natriumcarboxymethylstärken, Natriumstärkeglycolat sowie Mischungen hiervon. Ein erfindungsgemäß außerordentlich bevorzugtes anionisches Polysaccharid ist Aluminiumstärkeoctenylsuccinat.

Weitere besonders bevorzugte erfindungsgemäße Öl-in-Wasser-Emulsionen sind dadurch gekennzeichnet, dass das nichtionische Polysaccharid ausgewählt ist aus Stärken, Stärkehydrolysaten, Cellulose, Methylcellulose, Hydroxypropylcellulose, Hydroxyethylcellulose, Hydroxypropylmethylcellulose, Hydroxypropylethylcellulose, Hydroxyethylmethylcellulose sowie Mischungen hiervon.

Weitere besonders bevorzugte erfindungsgemäße Öl-in-Wasser-Emulsionen sind dadurch gekennzeichnet, dass das mindestens eine Polysaccharid in einer Gesamtmenge von 0,05 - 0,2 und besonders bevorzugt 0,09 - 0,2 Gew.-%, jeweils bezogen auf das Gesamtgewicht der Emulsion, enthalten ist. Es war besonders überraschend, dass schon mit relativ geringen Mengen an Polysaccharid ein schnelleres Trocknen der Emulsion auf der Haut erzielt werden konnte.

Die erfindungsgemäßen Öl-in-Wasser-Emulsionen zeichnen sich gegenüber dem Stand der Technik weiterhin durch einen geringen Anteil an einer Öl- oder Fettphase von 0,5 - 6,5 Gew.-%, bezogen auf das Gewicht der gesamten Emulsion, aus. Der geringe Anteil an dispergierter Öl- oder Fettphase führt zu einem verbesserten, nicht-fettenden Hautgefühl. Weiterhin stellen die erfindungsgemäßen Emulsionen eine hervorragende, nicht-komedogene Basis insbesondere für kosmetische und dermatologische Wirkstoffe dar, die zur Behandlung fettiger, unreiner Haut und/oder Akne-Haut bestimmt sind. Weiterhin stellen die erfindungsgemäßen Emulsionen eine hervorragende Basis für Sonnenschutzzusammensetzungen dar, da gerade der Fett- und Emulgatorgehalt häufig für Unverträglichkeitsreaktionen solcher Zusammensetzungen unter Einwirkung des Sonnenlichtes verantwortlich ist. Mit den erfindungsgemäßen Emulsionen lässt sich das Risiko für Unverträglichkeitsreaktionen deutlich minimieren. Weiterhin stellen die erfindungsgemäßen Emulsionen eine hervorragende Basis für selbstbräunende Zusammensetzungen dar, deren Wirkstoffe, insbesondere Dihydroxyaceton, in bekannten Emulsionen nur schwierig zu stabilisieren sind, da sie mit zahlreichen, üblicherweise eingesetzten Emulsionsbestandteilen undefinierte Reaktionen eingehen, die zur Deaktivierung des Wirkstoffes und zur Verfärbung des Kosmetikums führen. Weiterhin bietet eine solche Emulsion auch ökonomische Vorteile.
Allerdings lassen sich derartige Emulsionen üblicherweise nicht mit einer Viskosität herstellen, die für eine Applikation mit einem Kugel- oder Roll-on-Applikator erforderlich ist. Eine besondere Herausforderung der vorliegenden Erfindung war es daher, kosmetische Öl-in-Wasser-Emulsionen mit einem Anteil an einer Öl- oder Fettphase von 0,5 - 6,5 Gew.-%, bezogen auf das Gewicht der gesamten Emulsion, und einer für die Applikation als Roll-on ausreichenden Viskosität herzustellen. Zur Öl- oder Fettphase zählen erfindungsgemäß neben der mindestens einen bei 20 °C (unter Normalbedingungen) flüssigen Ölkomponente, die ausgewählt ist aus linearen und verzweigten gesättigten ein- oder mehrwertigen C₃ - C₃₀-Alkanolen, die mit mindestens einer Propylenoxid-Einheit pro Molekül verethert sind, Propylenglycolmonoestern von verzweigten gesättigten C₆ - C₃₀-Alkancarbonsäuren und verzweigten gesättigten C₁₀ - C₃₀-Alkanolen, auch Duftstoffe, sofern vorhanden. Darüber hinaus können auch bei 20 °C (unter Normalbedingungen) feste oder pastöse Fettkomponenten enthalten sein. Die Emulgatoren zählen definitionsgemäß nicht zur Öl- oder Fettphase.
Zu den bei 20 °C (unter Normalbedingungen) flüssigen Ölkomponenten, die ausgewählt sind aus linearen und verzweigten gesättigten ein- oder mehrwertigen C₃ - C₃₀-Alkanolen, die mit mindestens einer Propylenoxid-Einheit pro Molekül verethert sind, zählen bevorzugt Propanol, Glycerin, Propylenglycol, Butanol, Butandiol, Pentanol, Caprinalkohol, Caprylalkohol, Caprylylalkohol, Laurylalkohol, Tridecylalkohol, Myristylalkohol, Palmitylalkohol, Cetylalkohol, Stearylalkohol, Arachidylalkohol und Behenylalkohol, die mit 1, 2, 3, 4, 5, 6, 7, 8, 9, 10, 11, 12, 13, 14, 15, 16, 17, 18, 19 oder 20 Propylenoxideinheiten verethert sind.

Bevorzugte erfindungsgemäße Öl-in-Wasser-Emulsionen sind dadurch gekennzeichnet, dass die Ölkomponente i) ausgewählt ist aus Anlagerungsprodukten von mindestens 6 Propylenoxid-Einheiten pro Molekül an ein- oder mehrwertige C₃₋₃₀-Alkanole, insbesondere an Butanol, Butandiol, Myristylalkohol und Stearylalkohol.

Besonders bevorzugte erfindungsgemäße schweißhemmende Öl-in-Wasser-Emulsionen sind dadurch gekennzeichnet, dass die Ölkomponente i) ausgewählt ist aus PPG-3-Myristylether (z. B. erhältlich als Handelsprodukt Witconol^{®} APM), PPG-13-Butylether, PPG-14-Butylether (z. B. erhältlich als Handelsprodukt Ucon Fluid^{®} AP), PPG-9-Butylether (z. B. erhältlich als Handelsprodukt Breox^{®} B25), PPG-10-Butandiol (z. B. erhältlich als Handelsprodukt Macol^{®} 57) und PPG-15-Stearylether (z. B. erhältlich als Handelsprodukt Arlamol^{®} E), sowie Mischungen hiervon.

Weitere bevorzugte erfindungsgemäße Öl-in-Wasser-Emulsionen sind dadurch gekennzeichnet, dass die Ölkomponente ii) ausgewählt ist aus Propylenglycolmonoestern von verzweigten gesättigten C₆ - C₃₀-Alkancarbonsäuren.
Erfindungsgemäß besonders bevorzugte Ölkomponenten ii) sind ausgewählt aus Propylenglycolmonoisostearat, Propylenglycolmonoisopalmitat, Propylenglycolmonoisobehenat, Propylenglycolmonoisoarachinat, Propylenglycolmonoisomyristat, Propylenglycolmonoisocaprat, Propylenglycolmonoisocaprinat und Propylenglycolmonoisocaprylat sowie Mischungen hiervon.

Weitere bevorzugte erfindungsgemäße Öl-in-Wasser-Emulsionen sind dadurch gekennzeichnet, dass die Ölkomponente iii) ausgewählt ist aus verzweigten gesättigten C₁₀ - C₃₀-Alkanolen. Erfindungsgemäß besonders bevorzugte Ölkomponenten iii) sind ausgewählt aus Isostearylalkohol, Isocetylalkohol, Isomyristylalkohol, Isotridecylalkohol, Isoarachidylalkohol, Isobehenylalkohol, Isocaprylalkohol, Isocaprinylalkohol, Isocaprylylalkohol, sowie Mischungen hiervon.

Weitere bevorzugte erfindungsgemäße Öl-in-Wasser-Emulsionen sind dadurch gekennzeichnet, dass die mindestens eine Ölkomponente, ausgewählt aus den oben genannten Gruppen i), ii) und iii), in einer Gesamtmenge von 0,1 - 6,5 Gew.-%, bevorzugt 0,3 - 5 Gew.-%, besonders bevorzugt 0,5 - 3 Gew.-% und außerordentlich bevorzugt 1 - 2 Gew.-%, jeweils bezogen auf das Gesamtgewicht der Emulsion, enthalten ist.

Weitere bevorzugte erfindungsgemäß erhältliche Öl-in-Wasser-Emulsionen sind dadurch gekennzeichnet, dass die Öl- oder Fettphase in einer Gesamtmenge von 0,7 - 5 Gew.-%, bevorzugt 1 - 4 Gew.-%, besonders bevorzugt 1,5 - 3 Gew.-% und außerordentlich bevorzugt 2 - 2,5 Gew.-%, jeweils bezogen auf das Gesamtgewicht der Emulsion, enthalten ist.

Überraschend wurde festgestellt, dass die Lagerstabilität der erfindungsgemäßen Öl-in-Wasser-Emulsionen durch den Zusatz von mindestens einem nichtionischen Emulgator mit einem HLB-Wert im Bereich von 3 - 6 weiter gesteigert werden kann. Derartige lipophile Emulgatoren stabilisieren normalerweise Wasser-in-Öl-Emulsionen.
Erfindungsgemäße Öl-in-Wasser-Emulsionen sind dadurch gekennzeichnet, dass mindestens ein nichtionischer Emulgator mit einem HLB-Wert im Bereich von 3 - 6 in einer Gesamtmenge von 1,8 - 3 Gew.-%, bezogen auf das Gesamtgewicht der Emulsion, enthalten ist.
Besonders bevorzugte nichtionische Emulgatoren mit einem HLB-Wert im Bereich von 3 - 6 sind ausgewählt aus Steareth, Ceteth, Myristeth, Laureth, Trideceth, Arachideth und Beheneth mit jeweils 1 - 4 Ethylenoxid-Einheiten pro Molekül. Außerordentlich bevorzugt sind Steareth-1, Steareth-2, Steareth-3, Ceteth-1, Ceteth-2, Ceteth-3, Myristeth-1, Myristeth-2, Laureth-1 Beheneth-2, Beheneth-3 und Beheneth-4, insbesondere Steareth-2.

Weitere erfindungsgemäß bevorzugte Öl-in-Wasser-Emulsionen sind dadurch gekennzeichnet, dass mindestens ein nichtionischer Emulgator mit einem HLB-Wert im Bereich von 3 - 6 in einer Gesamtmenge von 2 - 2,8 Gew.-% und bevorzugt 2,4 - 2,6 Gew.-%, jeweils bezogen auf das Gewicht der gesamten Emulsion, enthalten ist.

Weiterhin wurde überraschend festgestellt, dass die Lagerstabilität der erfindungsgemäßen Öl-in-Wasser-Emulsionen durch den Zusatz von mindestens einem nichtionischen Emulgator mit einem HLB-Wert im Bereich von 12 - 18 weiter gesteigert werden kann. Erfindungsgemäße Öl-in-Wasser-Emulsionen sind dadurch gekennzeichnet, dass mindestens ein nichtionischer Emulgator mit einem HLB-Wert im Bereich von 12 - 18, ausgewählt aus Steareth, Ceteth, Myristeth, Laureth, Trideceth, Arachideth und Beheneth mit jeweils 7 - 40 Ethylenoxid-Einheiten pro Molekül, in einer Gesamtmenge von 1 - 2 Gew.-%, bezogen auf das Gewicht der gesamten Emulsion, enthalten ist. Bevorzugte nichtionische Emulgatoren mit einem HLB-Wert im Bereich von 12 - 18 sind insbesondere Steareth-15, Steareth-20, Steareth-21, Arachideth-20, Arachideth-21, Beheneth-20, Beheneth-21, Ceteth-20, Ceteth-30, Ceteth-15 und Myristeth-15.
Weitere erfindungsgemäß bevorzugte Öl-in-Wasser-Emulsionen sind dadurch gekennzeichnet, dass mindestens ein nichtionischer Emulgator mit einem HLB-Wert im Bereich von 12 - 18 in einer Gesamtmenge von 1,2 - 1,8 Gew.-% und besonders bevorzugt 1,5 - 1,7 Gew.-%, jeweils bezogen auf das Gewicht der gesamten Emulsion, enthalten ist.

Weitere erfindungsgemäß bevorzugte Öl-in-Wasser-Emulsionen sind dadurch gekennzeichnet, dass als nichtionischer Emulgator mit einem HLB-Wert im Bereich von 3 - 6 Steareth-2 in Kombination mit Steareth-21 als nichtionischem Emulgator mit einem HLB-Wert im Bereich von 12 - 18 enthalten ist. Derartige Emulsionen zeichnen sich durch eine besonders günstige Lager- und Temperaturstabilität aus.

Erfindungsgemäße Öl-in-Wasser-Emulsionen sind dadurch gekennzeichnet, dass das Gewichtsverhältnis der Gesamtmenge an nichtionischen Emulgatoren mit einem HLB-Wert im Bereich von 3 - 6 und Gesamtmenge an nichtionischen Emulgatoren mit einem HLB-Wert im Bereich von 12 - 18 von 0,9 bis 3, bevorzugt 1,3 - 1,9 beträgt.

In der nachfolgenden Tabelle sind verschiedene Öl-in-Wasser-Emulgatoren und Wasser-in-Öl-Emulgatoren und ihre HLB-Werte zusammengestellt. Hierbei handelt es sich um dem Fachmann allgemein bekannte Emulgatoren, wie sie beispielsweise in Kirk-Othmer, "Encyclopedia of Chemical Technology", 3. Aufl., 1979, Band 8, Seiten 913 - 916, aufgelistet sind. Für ethoxylierte Produkte wird der HLB-Wert nach der Formel HLB = (100 - L) : 5 berechnet, wobei L der Gewichtsanteil der lipophilen Gruppen, das heißt der Fettalkyl- oder Fettacylgruppen, in den Ethylenoxidaddukten, ausgedrückt in Gewichtsprozent, ist. Die HLB-Werte können nach Griffin berechnet werden, wie es beispielsweise im RÖMPP Chemie Lexikon, insbesondere in der Online-Version von November 2003, und den dort unter dem Stichwort "HLB-System" zitierten Handbüchern von Fiedler, Kirk-Othmer und Janistyn dargestellt beziehungsweise tabelliert ist. Sofern es in der Literatur unterschiedliche Angaben zum HLB-Wert einer Substanz gibt, sollte derjenige HLB-Wert für die erfindungsgemäße Lehre genutzt werden, der dem nach Griffin berechneten Wert am nächsten kommt. Falls sich auf diese Art und Weise kein eindeutiger HLB-Wert ermitteln lässt, ist der HLB-Wert, den der Hersteller des Emulgators angibt, für die erfindungsgemäße Lehre zu nutzen. Falls auch dies nicht möglich ist, ist der HLB-Wert experimentell zu ermitteln.

| **HLB-Wert** | **Chemische Bezeichnung** |
|---|---|
| 1 | Triglyceride gesättigter Fettsäuren |
| | Glyceryltrioleat |
| 1,5 | Ethylenglycoldistearat |
| 1,6 | Pur-Cellinöl |
| 1,8 | Sorbitantrioleat |
| | Glycerindioleat |
| 2,1 | Sorbitantristearat |
| 2,4 | Propylenglycollactostearat |
| 2,7 | Glycerinmonooleat |
| | Sorbitdioleat |
| 2,8 | Glycerinmonostearat |
| | Propylenglycolmono-/distearat, nicht selbstemulgierend |
| 2,9 | Ethylenglycolmonostearat |
| 3,0 | Decaglycerindecaoleat |
| | Decaglycerindecastearat |
| | Generol 122 (Rapeseed Sterols) |
| | Sucrosedistearat |
| 3,1 | Decaglycerindecaoleat |
| | Glycerylmonoricinoleat |
| | Pentaerythritylmonostearat |
| | Pentaerythritylsesquioleat |
| 3,2 | Ethylenglycolmonodistearat, nicht selbstemulgierend |
| | Glycolstearat |
| 3,3 | Glycerinmonolaurat |
| 3,4 | Propylenglycolmonostearat |
| 3,5 | Ethylenglycolmonostearat |
| | Pentaerythritylmonooleat |
| | Polyethylenglycol(100)monooleat |
| 3,6 | Glycerinmono-/dioleat, nicht selbstemulgierend |
| | Monoethoxylaurylether |
| 3,7 | Sorbitansesquioleat (Dehymuls SSO) |
| 3,8 | Glycerinmonodistearat, nicht selbstemulgierend |
| | Polyethylenglycol(100)monostearat |
| | Diglycerinsesquioleat |
| | N,N-Dimethylcaproamid |
| | Pentaerythritmonotallat |
| | Propylenglycolmonolaurat |
| 4,0 | Decaglycerinoctaoleat |
| 4,3 | Sorbitanmonooleat (Dehymuls SMO) |
| | Diethylenglycolmonostearat |
| 4,4 | 1.2-Propylenglycolmonodistearat, selbstemulgierend |
| 4,5 | Glycerinmonostearatpalmitat (90 %), nicht selbstemulgierend |
| | Propylenglycolmonolaurat |
| 4,7 | Sorbitanmonostearat (Dehymuls SMS) |
| | Diethylenglycolmonooleat |
| 4,8 | Pentaerythritmonolaurat |
| 4,9 | Polyoxyethylen(2)oleylalkohol (Polyoxyethylen(2)oleylether) |
| | Polyoxyethylen(2)stearylalkohol (Polyoxyethylen(2)stearylether) |
| 5,0 | Ethylenglycolmonodistearat |
| | Generol 122 E 5 (PEG-5 Soy Sterol) |
| | Polyethylenglycol(100)monoricinoleat |
| | Polyethylenglycol(200)distearat |
| | Polyglyceryl-3-isostearate (z. B. Isolan GI 34 von Tego) |
| 5,9 | Polyethylenglycol(200)dilaurat |
| 6,0 | Decaglycerintetraoleat |
| | Polyethylenglycol(100)monolaurat |
| | Polyethylenglycol(200)dioleat |
| 6,1 | Diethylenglycolmonolaurat (Diglycollaurat) |
| 6,3 | Polyethylenglycol(300)dilaurat |
| 6,4 | Glycerinmonoricinoleat |
| | Glycerinsorbitanmonolaurat |
| 6,5 | Diethylenglycolmonolaurat |
| | Natriumstearoyl-2-lactylat |
| 6,7 | Sorbitanmonopalmitat |
| 6,8 | Glycerinmonococoat |
| | Glycerinmonolaurat |
| 7,0 | Polyoxyethylen(2)C₁₀-C₁₄-fettalkoholether, Laureth-2 (Dehydol LS 2) |
| | Saccharosedistearat |
| 7,2 | Polyethylenglycol(400)dioleat |
| | Saccharosedioleat |
| 7,4 | Polyethylenglycol(100)monolaurat |
| | Saccharosedipalmitat |
| 7,5 | Saccharosedipalmitat |
| 7,6 | Glycerinsorbitanlaurat |
| 7,8 | Polyethylenglycol(400)distearat |
| 7,9 | Polyethylenglycol(200)monostearat |
| | Polyoxyethylen(3)tridecylalkohol |
| 8-8,2 | Polyethylenglycol(400)distearat |
| 8,0 | Polyoxyethylen(3)C₁₀-C₁₄-fettalkoholether, Laureth-3 (Dehydol LS 3) |
| | N.N-Dimethyllauramid |
| | Natriumlauroyllactylat, Natriumlauroyl-2-lactylat |
| | Polyethylenglycol(200)monooleat |
| | Polyethylenglycol(220)monotallat |
| | Polyethylenglycol(1500)dioleat |
| | Polyoxyethylen(4)oleylalkohol |
| | Polyoxyethylen(4)stearylcetylether |
| 8,2 | Triglycerinmonooleat |
| 8,3 | Diethylenglycolmonolaurat |
| 8,4 | Polyoxyethylen(4)cetylether |
| | Polyoxyethylenglycol(400)dioleat |
| 8,5 | Natriumcaproyllactylat |
| | Polyethylenglycol(200)monostearat |
| | Sorbitanmonooleat |
| 8,6 | Sorbitanmonolaurat (Dehymuls SML) |
| | Polyethylenglycol(200)monolaurat |
| 8,8 | Polyoxyethylen(4)myristylether |
| | Polyethylenglycol (400)dioleat |
| 8,9 | Nonylphenol, polyoxyethyliert mit 4 Mol EO |
| 9,0 | Oleth-5 (z. B. Eumulgin O 5) |
| 9,2 - 9,7 | Polyoxyethylen(4)laurylalkohol (je nach Handelsprodukt, z. B. Brij 30, Dehydol LS 4) |
| 9,3 | Polyoxyethylen(4)tridecylalkohol |
| 9,6 | Polyoxyethylen(4)sorbitanmonostearat |
| 9,8 | Polyethylenglyco1(200)monolaurat |
| 10-11 | Polyethylenglycol(400)monooleat |
| 10,0 | Didodecyldimethylammoniumchlorid |
| 10,0 | Polyethylenglycol(200)monolaurat |
| | Polyethylenglycol(400)dilaurat |
| | Polyethylenglycol(600)dioleat |
| | Polyoxyethylen(4)sorbitanmonostearat |
| | Polyoxyethylen(5)sorbitanmonooleat |
| 10,2 | Polyoxyethylen(40)sorbitol hexaoleat |
| 10,4 - 10,6 | Polyoxyethylenglycol(600)distearat |
| 10,5 | Polyoxyethylen(20)sorbitantristearat |
| 10,6 | Saccharosemonostearat |
| 10,7 | Saccharosemonooleat |
| 11 - 11,4 | Polyethylenglycol(400)monooleat |
| 11,0 | Polyethylenglycol(350)monostearat |
| | Polyethylenglycol(400)monotallat |
| | Polyoxyethylenglycol(7)monostearat |
| | Polyoxyethylenglycol(8)monooleat |
| | Polyoxyethylen(20)sorbitantrioleat |
| | Polyoxyethylen(6)tridecylalkohol |
| 11,1 | Polyethylenglycol(400)monostearat |
| 11,2 | Polyoxyethylen(9)monostearat |
| | Saccharosemonooleat |
| | Saccharosemonostearat |
| 11,4 | Polyoxyethylen(50)sorbitol hexaoleat |
| | Saccharosemonotallat |
| | Saccharosestearatpalmitat |
| 11,6 | Polyoxyethylenglycol(400)monoricinoleat |
| 11,7 | Saccharosemonomyristat |
| | Saccharosemonopalmitat |
| 12,0 | PEG-10 Soy Sterol (z. B. Generol 122 E 10) |
| | Triethanolaminoleat |
| 12,2-12,3 | Nonylphenol, ethoxyliert mit 8 Mol EO |
| 12,2 | Saccharosemonomyristat |
| 12,4 | Saccharosemonolaurat |
| | Polyoxyethylen(10)oleylalkohol, Polyoxyethylen(10)oleylether |
| | Polyoxyethylen(10)stearylalkohol, Polyoxyethylen(10)stearylether |
| 12,5 | Polyoxyethylen(10)stearylcetylether |
| 12,7 | Polyoxyethylen(8)tridecylalkohol |
| 12,8 | Polyoxyethylenglycol(400)monolaurat |
| | Saccharosemonococoat |
| 12,9 | Polyoxyethylen(10)cetylether |
| 13 | Glycerinmonostearat, ethoxyliert (20 Mol EO) |
| 13,0 | Eumulgin O 10 (Polyoxyethylen(10)oleylether) |
| | Eumulgin 286 (Nonoxynol-10) |
| | Eumulgin B 1 (Ceteareth-12) |
| 13,0 | C12-Fettamine, ethoxyliert (5 Mol EO) |
| 13,1 | Nonylphenol, ethoxyliert (9,5 Mol EO) |
| 13,2 | Polyethylenglycol(600)monostearat |
| | Polyoxyethylen(16)tallöl |
| 13,3 | Polyoxyethylen(4)sorbitanmonolaurat |
| 13,5 | Nonylphenol, ethoxyliert (10,5 Mol EO) |
| | Polyethylenglycol(600)monooleat |
| 13,7 | Polyoxyethylen(10)tridecylalkohol |
| | Polyethylenglycol(660)monotallat |
| | Polyethylenglycol(1500)monostearat |
| | Polyoxyethylenglycol(1500)dioleat |
| 13,9 | Polyethylenglycol(400)monococoat |
| | Polyoxyethylen(9)monolaurat |
| 14-16 | Eumulgin HRE 40 (Ricinusöl, mit 40 EO ethoxyliert und hydriert) |
| 14,0 | Polyoxyethylen(12)laurylether |
| | Polyoxyethylen(12)tridecylalkohol |
| 14,2 | Polyoxyethylen(15)stearylalkohol |
| 14,3 | Polyoxyethylen(15)stearylcetylether |
| 14,4 | Gemisch aus C12-C15-Fettalkoholen mit 12 Mol EO |
| 14,5 | Polyoxyethylen(12)laurylalkohol |
| 14,8 | Polyoxyethylenglycol(600)monolaurat |
| 14,9 - 15,2 | Sorbitanmonostearat, mit 20 EO ethoxyliert (z. B. Eumulgin SMS 20) |
| 15 - 15,9 | Sorbitanmonooleat, mit 20 EO ethoxyliert (z. B. Eumulgin SMO 20) |
| 15,0 | PEG-20 Glyceryl stearate (z. B. Cutina E 24) |
| | PEG-40 Castor Oil (z. B. Eumulgin RO 40) |
| | Decylglucosid (Oramix NS 10) |
| | Dodecylglucosid (Plantaren APG 600) |
| | Dodecyltrimethylammoniumchlorid |
| | Nonylphenol, ethoxyliert mit 15 Mol EO |
| | Polyethylenglycol(1000)monostearat |
| | Polyoxyethylen(600)monooleat |
| 15-17 | Eumulgin HRE 60 (Ricinusöl, mit 60 EO ethoxyliert und hydriert) |
| 15,3 | C12-Fettamine, polyoxyethyliert mit 12 Mol EO |
| | Polyoxyethylen(20)oleylalkohol, Polyoxyethylen(20)oleylether |
| 15,4 | Polyoxyethylen(20)stearylcetylether (z. B. Eumulgin B 2 (Ceteareth-20)) |
| 15,5 | Polyoxyethylen(20)stearylalkohol |
| 15,6 | Polyoxyethylenglycol(1000)monostearat |
| | Polyoxyethylen(20)sorbitanmonopalmitat |
| 15,7 | Polyoxyethylen(20)cetylether |
| 15,9 | Dinatriumtriethanolamindistearylheptaglycolethersulfosuccinat |
| 16,0 | Nonylphenol ethoxyliert mit 20 Mol EO |
| | Polyoxyethylen(25)propylenglycolstearat |
| 16 - 16,8 | Polyoxyethylen(30)monostearat |
| 16,3-16,9 | Polyoxyethylen(40)monostearat |
| 16,5 - 16,7 | Polyoxyethylen(20)sorbitanmonolaurat (z. B. Eumulgin SML 20) |
| 16,6 | Polyoxyethylen(20)sorbit |
| 16,7 | C18-Fettamine, polyoxyethyliert mit 5 Mol EO |
| | Polyoxyethylen(23)laurylalkohol |
| 17,0 | Ceteareth-30, z. B. Eumulgin B 3 |
| | Octylglucosid (Triton CG 110) |
| | Polyoxyethylen(30)glycerylmonolaurat |
| 17,1 | Nonylphenol, ethoxyliert mit 30 Mol EO |
| 17,4 | Polyoxyethylen(40)stearylalkohol |

Weitere erfindungsgemäß besonders bevorzugte Öl-in-Wasser-Emulsionen sind dadurch gekennzeichnet, dass Steareth-2, Steareth-21 und PPG-15-Stearylether enthalten sind. Derartige Emulsionen zeichnen sich durch eine besonders hohe Lager- und Temperaturstabilität aus und tragen gleichzeitig zu einem verbesserten, nicht-klebrigen Hautgefühl bei.
Erfindungsgemäß außerordentlich bevorzugte Öl-in-Wasser-Emulsionen sind dadurch gekennzeichnet, dass neben Steareth-2, Steareth-21 und PPG-15-Stearylether Aluminiumstärkeoctenylsuccinat als Polysaccharid enthalten ist. Dieses Polysaccharid ist beispielsweise unter den Handelsnamen Dry Flo und Dry Flo Plus von National Starch erhältlich. Weitere erfindungsgemäß außerordentlich bevorzugte Öl-in-Wasser-Emulsionen sind dadurch gekennzeichnet, dass neben Steareth-2, Steareth-21 und PPG-15-Stearylether mindestens ein Distärkephosphat enthalten ist. Dieses Polysaccharid ist beispielsweise unter dem Handelsnamen Maize PO 4 PH "B" von Agrana erhältlich. Derartige Emulsionen zeichnen sich durch eine besonders hohe Lager- und Temperaturstabilität, ein hervorragendes, nicht-klebriges Hautgefühl und optimale Trocknungseigenschaften auf der Haut aus.

Der Anteil des Wassers an der erfindungsgemäßen Zusammensetzung beträgt mindestens 60 Gew.-%, bevorzugt 65 bis 90 Gew.-%, besonders bevorzugt 70 - 85 Gew.-%, außerordentlich bevorzugt 75 - 80 Gew.%, jeweils bezogen auf die Gesamtzusammensetzung.

Weitere erfindungsgemäß bevorzugte Öl-in-Wasser-Emulsionen sind dadurch gekennzeichnet, dass insgesamt maximal 3 Gew.-%, bevorzugt maximal 1 Gew.-% und besonders bevorzugt 0 Gew.-%, jeweils bezogen auf das Gewicht der gesamten Emulsion, an einwertigen C₁ - C₃-Alkanolen, wie Ethanol oder Isopropanol, enthalten ist. Die erfindungsgemäßen Emulsionen können unter bestimmten Bedingungen durch einen Zusatz an Ethanol oder Isopropanol, insbesondere in höheren Mengen, beispielsweise 5 Gew.-% und mehr, in ihrer Lager- und/oder Temperaturstabilität destabilisiert werden.

Die erfindungsgemäßen Öl-in-Wasser-Emulsionen wurden insbesondere für Roll-on-Produkte entwickelt, das heißt, für die Applikation mit einem Kugelapplikator oder Roll-on-Applikator. Für optimale Dosiereigenschaften darf die Emulsion weder zu niedrig-viskos noch zu hoch-viskos sein. Erfindungsgemäße Öl-in-Wasser-Emulsionen sind daher durch eine Viskosität im Bereich von 1000 - 5000 mPas, bevorzugt 1500 - 4000 mPas und besonders bevorzugt 1700 - 2200 mPas gekennzeichnet. Diese Viskositätsangaben beziehen sich auf Messungen mit einem Brookfield-Viskosimeter, die 1 Tag nach Herstellung der Emulsion mit Spindel RV 4, bei einer Scherrate (Umdrehungszahl der Spindel) von 20 s⁻¹ ohne Helipath bei einer Umgebungstemperatur und einer Probentemperatur von jeweils 20 °C durchgeführt werden.

### Schweißhemmende Wirkstoffe

Erfindungsgemäße Öl-in-Wasser-Emulsionen sind dadurch gekennzeichnet, dass der kosmetische Wirkstoff c) ausgewählt ist aus schweißhemmenden Wirkstoffen. Erfindungsgemäß bevorzugte schweißhemmende oder Antitranspirant-Wirkstoffe sind ausgewählt aus den wasserlöslichen adstringierenden anorganischen und organischen Salzen des Aluminiums, Zirkoniums und Zinks bzw. beliebigen Mischungen dieser Salze, enthalten ist. Besonders bevorzugte Antitranspirant-Wirkstoffe sind ausgewählt aus den Aluminiumchlorhydraten, insbesondere den Aluminiumchlorhydraten mit der allgemeinen Formel [Al₂(OH)₅Cl · 2-3 H₂O]ₙ, die in nicht-aktivierter oder in aktivierter (depolymerisierter) Form vorliegen können, weiterhin Aluminiumsesquichlorohydrat, Aluminiumchlorhydrex-Propylenglykol (PG) oder -Polyethylenglykol (PEG), Aluminiumsesquichlorhydrex-PG oder -PEG, Aluminium-PG-dichlorhydrex oder Aluminium-PEG-dichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirkonium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Kaliumaluminiumsulfat (KAI(SO₄)₂ · 12 H₂O, Alaun), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Komplexe von Lanthan und Cer, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat, Zinksulfat und Zirkoniumchlorohydrat. Erfindungsgemäß wird unter Wasserlöslichkeit eine Löslichkeit von wenigstens 5 Gew.-% bei 20 °C verstanden. 5 Gew.-% bedeutet, dass 5 g des Antitranspirant-Wirkstoffes in 95 g Wasser bei 20 °C löslich sind. Die Antitranspirant-Wirkstoffe werden erfindungsgemäß bevorzugt als wässrige Lösungen eingesetzt. Bei Verwendung von Zirconiumsalzen und Aluminium-Zirconium-Salzen ist zu beachten, dass die vorgefertigten wässrigen Wirkstofflösungen möglichst frisch zubereitet sind. Bei längerer Lagerzeit können die Zirconiumverbindungen zur Polymerisation neigen, was sowohl mit einem Aktivitätsverlust als auch mit einer Zunahme der Viskosität einhergeht.
Besonders bevorzugte erfindungsgemäße schweißhemmende Emulsionen sind dadurch gekennzeichnet, dass der mindestens eine Antitranspirant-Wirkstoff in einer Menge von 1 - 38 Gew.-%, vorzugsweise 5 - 25 Gew.-% und insbesondere 10 - 20 Gew.-%, enthalten ist, jeweils bezogen auf das Gesamtgewicht der Aktivsubstanz in der Gesamtzusammensetzung. In einer besonders bevorzugten Ausführungsform enthält die Zusammensetzung ein adstringierendes Aluminiumsalz, insbesondere Aluminiumchlorohydrat, das beispielsweise in Form einer wässrigen Lösung als Locron^{®} L von Clariant, als Chlorhydrol^{®} sowie in aktivierter Form als Reach^{®} 501 von Reheis vertrieben wird. Unter der Bezeichnung Reach^{®} 301 wird ein Aluminiumsesquichlorohydrat von Reheis angeboten, das ebenfalls besonders bevorzugt ist. Auch die Verwendung von Aluminium-Zirkonium-Tetrachlorohydrex-Glycin-Komplexen, die beispielsweise von Reheis unter der Bezeichnung Rezal^{®} 36G im Handel sind, kann erfindungsgemäß besonders bevorzugt sein.

### Desodorierende Wirkstoffe

Erfindungsgemäß bevorzugte Öl-in-Wasser-Emulsionen sind dadurch gekennzeichnet, dass ein zusätzlicher kosmetischer Wirkstoff c) ausgewählt ist aus desodorierenden Wirkstoffen. Erfindungsgemäß bevorzugte desodorierende Wirkstoffe sind Geruchsabsorber, desodorierend wirkende Ionenaustauscher, keimhemmende Mittel, präbiotisch wirksame Komponenten sowie Enzyminhibitoren oder, besonders bevorzugt, Kombinationen der genannten Wirkstoffe. Silicate dienen als Geruchsabsorber, die auch gleichzeitig die rheologischen Eigenschaften der erfindungsgemäßen Zusammensetzung vorteilhaft unterstützen. Zu den erfindungsgemäß besonders vorteilhaften Silicaten zählen vor allem Schichtsilicate und unter diesen insbesondere Montmorillonit, Kaolinit, Ilit, Beidellit, Nontronit, Saponit, Hectorit, Bentonit, Smectit und Talkum. Weitere vorteilhafte Geruchsabsorber sind beispielsweise Zeolithe, Zinkricinoleat, Cyclodextrine, bestimmte Metalloxide, wie z. B. Aluminiumoxid, sowie Chlorophyll. Sie werden bevorzugt in einer Menge von 0,1 - 10 Gew.-%, besonders bevorzugt 0,5 - 7 Gew.-% und außerordentlich bevorzugt 1 - 5 Gew.-%, jeweils bezogen auf die Gesamtzusammensetzung, eingesetzt.
Als keimhemmende oder antimikrobielle Wirkstoffe erfindungsgemäß bevorzugt sind insbesondere Organohalogenverbindungen sowie -halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzedoniumchlorid. Weiterhin sind Phenol, Phenoxyethanol, Zinklactat, Dinatriumdihydroxyethylsulfosuccinylundecylenat, Natriumbicarbonat, Natriumphenolsulfonat und Zinkphenolsulfonat, Ketoglutarsäure, Terpenalkohole wie z. B. Farnesol, Chlorophyllin-Kupfer-Komplexe, α-Monoalkylglycerinether mit einem verzweigten oder linearen gesättigten oder ungesättigten, gegebenenfalls hydroxylierten C₆ - C₂₂-Alkylrest, besonders bevorzugt α-(2-Ethylhexyl)glycerin-ether, im Handel erhältlich als Sensiva^{®} SC 50 (ex Schülke & Mayr), Carbonsäureester des Mono-, Di- und Triglycerins (z. B. Glycerinmonolaurat, Diglycerinmonocaprinat), Lantibiotika sowie Pflanzenextrakte (z. B. grüner Tee und Bestandteile des Lindenblütenöls) als keimhemmende Wirkstoffe bevorzugt.
Weitere bevorzugte Deodorant-Wirkstoffe sind ausgewählt aus sogenannten präbiotisch wirksamen Komponenten, worunter erfindungsgemäß solche Komponenten zu verstehen sind, die nur oder zumindest überwiegend die geruchsbildenden Keime der Hautmikroflora hemmen, nicht aber die erwünschten, das heißt, die nicht-geruchsbildenden Keime, die zu einer gesunden Hautmikroflora gehören. Explizit sind hier die Wirkstoffe, die in den Offenlegungsschriften DE 10333245 und DE 10 2004 011 968 als präbiotisch wirksam offenbart sind, mit einbezogen; dazu gehören Nadelbaumextrakte, insbesondere aus der Gruppe der Pinaceae, und Pflanzenextrakte aus der Gruppe der Sapindaceae, Araliaceae, Lamiaceae und Saxifragaceae, insbesondere Extrakte aus *Picea spp., Paullinia sp*., *Panax sp., Lamium album* oder *Ribes nigrum* sowie Mischungen dieser Substanzen.
Weitere bevorzugte Deodorant-Wirkstoffe sind ausgewählt aus den keimhemmend wirkenden Parfümölen und den Deosafe-Parfümölen, die von der Firma Symrise, vormals Haarmann und Reimer, erhältlich sind.
Zu den Enzyminhibitoren gehören im Sinne der vorliegenden Erfindung Stoffe, die die für die Schweißzersetzung verantwortlichen Enzyme, insbesondere die Arylsulfatase, β-Glucuronidase, Aminoacylase, Esterasen, Lipasen und/oder Lipoxigenase, hemmen, z. B. bevorzugt Trialkylcitronensäureester, insbesondere Triethylcitrat, oder Zinkglycinat. Weitere Inhibitorsubstanzen der für die Schweißzersetzung verantwortlichen Enzyme und Keime, beispielsweise Arylsulfatase, β-Glucuronidase, Aminoacylase, Esterasen, Lipasen und/oder Lipoxigenase, sind offenbart in WO 03/039505 A2, WO 01/99376 A2, EP 1430879 A2, EP 1428520 A2, EP 1738803 A1, EP 1576946 A1 und DE 10216368 A1.
Weitere erfindungsgemäß bevorzugte Emulsionen sind dadurch gekennzeichnet, dass mindestens ein desodorierender Wirkstoff in einer Gesamtmenge von 0,1 - 10 Gew.-%, bevorzugt 0,2 - 7 Gew.-%, besonders bevorzugt 0,3 - 5 Gew.-% und außerordentlich bevorzugt 0,4 - 1,0 Gew.-%, jeweils bezogen auf das Gesamtgewicht der Aktivsubstanz in der Gesamtzusammensetzung, enthalten ist.

Weitere erfindungsgemäß bevorzugte Emulsionen sind dadurch gekennzeichnet, dass mindestens ein zusätzlicher kosmetischer Wirkstoff c), ausgewählt aus Monomeren, Oligomeren oder Polymeren von Aminosäuren, N-C₂-C₂₄-Acylaminosäuren und/oder den Estern und/oder den physiologisch verträglichen Salzen dieser Substanzen, enthalten ist. Viele dieser Wirkstoffe werden als Antiageing-Wirkstoffe eingesetzt und/oder wirken günstig auf den Feuchtigkeitshaushalt der Haut ein und/oder haben eine hautberuhigende Wirkung.
Die Monomere der Aminosäuren und/oder der N-C₂-C₂₄-Acylaminosäuren sind bevorzugt ausgewählt aus Alanin, Arginin, Asparagin, Asparaginsäure, Canavanin, Citrullin, Cystein, Cystin, Dipalmitoylhydroxyprolin, Desmosin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, Zink- und Natriumpyroglutamat und Natriumlauroylglutamat.
Der C₂ - C₂₄-Acylrest, mit dem die Aminosäuren, insbesondere die genannten bevorzugten Aminosäuren, an der Aminogruppe derivatisiert sind, ist bevorzugt ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten.
Mit den vorgenannten C₂ - C₂₄-Acylresten können die Aminosäuren, die eine OH-Gruppe tragen, auch an dieser OH-Gruppe verestert sein. Ein erfindungsgemäß bevorzugtes Beispiel hierfür ist Hydroxyprolin, das mit zwei, bevorzugt linearen, C₂-C₂₂-Fettsäureresten N-acyliert und verestert ist, besonders bevorzugt Dipalmitoylhydroxyprolin, das z. B. unter der Bezeichnung Sepilift DPHP von der Firma Seppic erhältlich ist.
Die physiologisch verträglichen Salze der erfindungsgemäß bevorzugten Aminosäuren oder Aminosäurederivate sind ausgewählt aus den Ammonium-, Alkalimetall-, Magnesium-, Calcium-, Aluminium-, Zink- und Mangan-Salzen. Besonders bevorzugt sind die Natrium-, Kalium-, Magnesium-, Aluminium-, Zink- und Mangan-Salze.
Unter Aminosäureoligomeren werden erfindungsgemäß Peptide mit 2 - 30, bevorzugt 2 - 15, Aminosäuren, verstanden. Die Oligomere der Aminosäuren und/oder der N-C₂-C₂₄-Acylaminosäuren sind bevorzugt ausgewählt aus Di-, Tri-, Tetra-, Penta-, Hexa- oder Pentadecapeptiden, die acyliert und/oder verestert sein können. Zahlreiche dieser Aminosäureoligomere stimulieren die Collagensynthese beziehungsweise sind in der Lage, Zellen des Immunsystems, wie Mastzellen und Makrophagen, zu rekrutieren, die dann über die Freisetzung von Wachstumsfaktoren Reparaturprozesse im Gewebe, z.B. die Collagensynthese, induzieren, beziehungsweise sind in der Lage, an die Sequenz Arg-Phe-Lys in Thrombospondin I (TSP-1) zu binden und damit aktives TGF-ß (*tissue growth factor*), der die Synthese von Collagen in dermalen Fibroblasten induziert, freizusetzen. Derartige Aminosäureoligomere werden bevorzugt als Wirkstoffe gegen die Hautalterung verwendet.
Erfindungsgemäß bevorzugte, gegebenenfalls N-acylierte und/oder veresterte Dipeptide sind Acetyl-Citrullyl-Arginin (z. B. Exsy-Algine von Exsymol mit der INCI-Bezeichnung Acetyl Citrull Amido Arginine), Tyr-Arg (Dipeptide-1), Val-Trp (Dipeptide-2), Asn-Phe, Asp-Phe, N-Palmitoyl-ß-Ala-His, N-Acetyl-Tyr-Arg-hexyldecylester (z. B. Calmosensine von Sederma), Carnosin (ß-Ala-His) und N-Palmitoyl-Pro-Arg. Erfindungsgemäß bevorzugte, gegebenenfalls N-acylierte und/oder veresterte Tripeptide sind Gly-His-Lys, das z. B. unter der Bezeichnung "Omega-CH-Aktivator" von der Firma GfN oder in acylierter Form (N-Palmitoyl-Gly-His-Lys) unter der Bezeichnung Biopeptide CL von Sederma erhältlich ist, aber (in acylierter Form) auch einen Bestandteil des Produktes Matrixyl 3000 von Sederma darstellt. Das Tripeptid Gly-His-Lys kann auch als Kupfersalz (Cu²⁺) eingesetzt werden und ist als solches über ProCyte Corporation zu beziehen. Weiterhin können Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und Ile geeignet. Die erfindungsgemäß bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Orn, oder Citrullin ersetzt. Ein weiteres erfindungsgemäß bevorzugtes Tripeptid ist Gly-His-Arg (INCI-Bezeichnung: Tripeptide-3) sowie dessen Derivat N-Myristoyl-Gly-His-Arg, das z. B. unter der Bezeichnung Collasyn 314-GR von Therapeutic Peptide Inc. erhältlich ist; weitere erfindungsgemäß bevorzugte Tripeptide sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure), Dap-Val-Lys, Palmitoyl-Lys-Val-Lys, z. B. erhältlich von der Firma Pentapharm unter der Bezeichnung SYN^{®}-COLL, Lys-Pro-Val, Tyr-Tyr-Val, Tyr-Val-Tyr, Val-Tyr-Val (Tripeptide-2), Tripeptide-4 (z. B. ATPeptide, zu beziehen über IMPAG), His-Ala-Orn N-Elaidoyl-Lys-Phe-Lys und N-Acetyl-Arg-Lys-Arg-NH₂.
Erfindungsgemäß bevorzugte, gegebenenfalls N-acylierte und/oder veresterte Tetrapeptide sind ausgewählt aus Rigin und Rigin-basierten Tetrapeptiden sowie ALAMCAT-Tetrapeptiden. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg, das z. B. unter der Bezeichnung Eyeliss von Sederma erhältlich ist, aber auch einen Bestandteil des Produktes Matrixyl 3000 von Sederma darstellt. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im folgenden definiert ist, ausgewählt werden. Besonders bevorzugte Rigin-basierte Tetrapeptide umfassen: Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus Gln und den Aminosäuren, die Gln substituieren können, Xcc ausgewählt ist aus Pro oder Arg und den Aminosäuren, die Pro und Arg substituieren können.
Die bevorzugten Aminosäuren, die Gly ersetzen können, beinhalten eine aliphatische Seitenkette, z. B. β-Ala, Ala, Val, Leu, Pro, Sarcosin (Sar) und Isoleucin (Ile).
Die bevorzugten Aminosäuren, die Gln ersetzen können, beinhalten eine Seitenkette mit einer Aminogruppe, die bei neutralem pH (pH 6-7) überwiegend ungeladen vorliegt, z.B. Asn, Lys, Orn, 5-Hydroxyprolin, Citrullin und Canavanin.
Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin.
Als Rigin-Analoga sind erfindungsgemäß Gly-Gln-Arg-Pro und Val-Val-Arg-Pro bevorzugt.
ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (Ile). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z.B. Gln, Asn, Lys, Orn, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt. Erfindungsgemäß bevorzugte, gegebenenfalls N-acylierte und/oder veresterte Pentapeptide sind ausgewählt aus Lys-Thr-Thr-Lys-Ser und seinen N-acylierten Derivaten, besonders bevorzugt N-Palmitoyl-Lys-Thr-Thr-Lys-Ser, das unter der Bezeichnung Matrixyl von der Firma Sederma erhältlich ist, weiterhin N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Val-Gly-Val-Ala-Pro-Gly und seine N-acylierten Derivate, besonders bevorzugt N-Palmitoyl-Val-Gly-Val-Ala-Pro-Gly, das unter der Bezeichnung Biopeptide EL von der Firma Sederma erhältlich ist, weiterhin Acetyl-Hexapeptide-3 (Argireline von Lipotec), Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience) und Hexapeptide-10 (z. B. Collaxyl von Vincience oder Seriseline von Lipotec), Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), Ala-Asp-Leu-Lys-Pro-Thr (Hexapeptide-3, z. B. Peptide 02 von Vincience), Val-Val-Arg-Pro-Pro-Pro, Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Ala-Arg-His-Methylnorleucin-Homophenylalanin-Trp (Hexapeptide-7), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience), Hexapeptide-10 (z. B. Collaxyl von Vincience oder Seriseline von Lipotec) und Hexapeptide-11 (z. B. Peptamide-6 von Arch Personal Care). Ein erfindungsgemäß bevorzugtes Pentadecapeptid ist z. B. der Rohstoff Vinci 01 von Vincience (Pentadecapeptide-1). Ein weiteres bevorzugtes Aminosäureoligomer ist das Peptidderivat L-Glutamylaminoethyl-indol (Glistin von Exsymol).
Erfindungsgemäß besonders bevorzugt ist die Kombination aus N-Palmitoyl-Gly-His-Lys und N-Palmitoyl-Gly-Gln-Pro-Arg, wie sie beispielsweise in dem Rohstoff Matrixyl 3000 von der Firma Sederma erhältlich ist.

Die Polymere der Aminosäuren und/oder der N-C₂-C₂₄-Acylaminosäuren sind bevorzugt ausgewählt aus pflanzlichen und tierischen Proteinhydrolysaten und/oder Proteinen. Tierische Proteinhydrolysate sind z. B. Elastin-, Collagen-, Keratin-, Seiden- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin^{®} (Diamalt), Gluadin^{®} (Cognis), Lexein^{®} (Inolex) und Crotein^{®} (Croda). Besonders bevorzugt sind Sojaproteinhydrolysate, z. B. die Handelsprodukte Phytokine von Coletica oder Ridulisse C von Silab. Proteinhydrolysate können naturgemäß auch monomere Aminosäuren und Oligopeptide enthalten; ihre Zusammensetzung ist normalerweise nicht definiert.
Ebenfalls möglich ist der Einsatz von Acylderivaten der Proteinhydrolysate, z. B. in Form ihrer Fettsäure-Kondensationsprodukte. Entsprechende Handelsprodukte sind z. B. Lamepon^{®} (Cognis), Gluadin^{®} (Cognis), Lexein^{®} (Inolex), Crolastin^{®} oder Crotein^{®} (Croda).
Erfindungsgemäß bevorzugt sind auch kationisierte Proteinhydrolysate. Besonders bevorzugt sind kationische Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Weiterhin können die kationischen Proteinhydrolysate auch noch weiter derivatisiert sein. Als typische Beispiele für erfindungsgemäß verwendete kationische Proteinhydrolysate und -derivate sind einige der unter den INCI- Bezeichnungen im "International Cosmetic Ingredient Dictionary and Handbook", (seventh edition 1997, The Cosmetic, Toiletry, and Fragrance Association, Washington, DC) genannten und im Handel erhältlichen Produkte aufgeführt: Cocodimonium Hydroxypropyl Hydrolyzed Collagen, Steardimonium Hydroxypropyl Hydrolyzed Collagen, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/ Myristyl Ether HCl. Außerordentlich bevorzugt sind die kationischen Proteinhydrolysate und -derivate auf pflanzlicher Basis.

In einer weiteren bevorzugten Ausführungsform sind die Polymeren der Aminosäuren ausgewählt aus DNA-Reparaturenzymen.
Erfindungsgemäß bevorzugte DNA-Reparaturenzyme sind Photolyase und T4 Endonuclease V, letztere im weiteren mit "T4N5" abgekürzt. Diese beiden Enzyme sind im Stand der Technik bereits als sogenannte DNA-Reparatur-Enzyme bekannt. Unter DNA-Reparatur ist definitionsgemäß die Spaltung bzw. Entfernung von UV-induzierten Pyrimidindimeren aus der DNA zu verstehen. Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans*, einem phototrophen marinen Mikroorganismus. Die Photolyase aus A. *nidulans* wird in technisch relevanten Mengen mittlerweile aus E. coli gewonnen. Photolyase ist zur Aktivierung auf Licht angewiesen.
Das Enzym T4 Endonuclease V wird vom *den*V-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv.
Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosome™, liposomenverkapselte T4N5 z. B. unter der Bezeichnung Ultrasome™ von der Firma AGI Dermatics, USA, erhältlich.
Bevorzugte erfindungsgemäße Emulsionen sind dadurch gekennzeichnet, dass sie mindestens einen der Rohstoffe Photosome™ oder Ultrasome™ in einer Gesamtmenge von 0,1 - 10 Gew.-%, bevorzugt 0,5 - 5,0 Gew.-% und besonders bevorzugt 1,0 - 4,0 Gew.-%, jeweils bezogen auf die gesamte Emulsion, enthalten.
Bevorzugte erfindungsgemäße Emulsionen sind dadurch gekennzeichnet, dass sie mindestens ein Monomer, Oligomer oder Polymer von Aminosäuren, N-C₂-C₂₄-Acylaminosäuren und/oder den Estern und/oder den physiologisch verträglichen Salzen dieser Substanzen in einer Gesamtmenge von 0,01 - 10 Gew.-%, bevorzugt 0,1 - 5 Gew.-% und besonders bevorzugt 0,1 - 3 Gew.%, jeweils bezogen auf die gesamte Emulsion, enthalten.

In einer weiteren bevorzugten Ausführungsform liegen die Monomeren, Oligomeren und Polymeren von Aminosäuren, N-C₂-C₂₄-Acylaminosäuren, den Estern und/oder den physiologisch verträglichen Salzen dieser Substanzen in geträgerter Form vor, insbesondere aufgetragen auf feinteiligen, pulverförmigen Substraten wie Kieselgel, insbesondere Aerosil-Typen, Talkum, modifizierten Stärken und Stärkederivaten, kristalliner Cellulose, Cellulosepulvern, Lactoglobulinderivaten, Microsponges, Polymerpartikeln aus Nylon, Polyolefinen, Polycarbonaten, Polyurethanen, Polyacrylaten, (Meth)acrylat- oder (Meth)acrylat-Vinyliden-Copolymeren, die vernetzt sein können, Polyestern, Polyamiden, Polystyrolen, Teflon und Siliconen. Ein besonders bevorzugter Rohstoff dieser Art sind die Vegetal Filling Spheres von Coletica.
Erfindungsgemäß besonders bevorzugte kosmetische Emulsionen sind dadurch gekennzeichnet, dass sie mindestens einen zusätzlichen kosmetischen Wirkstoff c), der ausgewählt ist aus Monomeren, Oligomeren und Polymeren von Aminosäuren, N-C₂-C₂₄-Acylaminosäuren und/oder den Estern und/oder den physiologisch verträglichen Salzen dieser Substanzen, in einer Gesamtmenge von 0,000001 - 5 Gew.-%, bevorzugt 0,00001 - 2 Gew.-%, besonders bevorzugt 0,0001 - 1 Gew.-% und außerordentlich bevorzugt 0,005 - 0,5 Gew.-%, jeweils bezogen auf den Gehalt an Aktivsubstanz in der gesamten Emulsion, enthalten.
In einer weiteren bevorzugten Ausführungsform enthalten die erfindungsgemäßen Öl-in-Wasser-Emulsionen als zusätzlichen kosmetischen Wirkstoff c) mindestens ein DNA-Oligonucleotid oder ein RNA-Oligonucleotid. Diesen Komponenten werden positive Effekte insbesondere bei der Anti-Falten- und der Anti-Ageing-Behandlung zugeordnet.
Erfindungsgemäß werden unter einem Oligonucleotid Polymerisate aus 2 bis 20, bevorzugt 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glykosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5'-triphosphat) und GTP (Guanosin-5'-triphosphat).
Ein erfindungsgemäß besonders bevorzugtes Oligonucleotid ist das Thymidin-Dinucleotid.
Bevorzugte erfindungsgemäße Öl-in-Wasser-Emulsionen sind dadurch gekennzeichnet, dass sie mindestens ein DNA-Oligonucleotid oder RNA-Oligonucleotid in einer Gesamtmenge von 0,0001 - 5 Gew.-%, bevorzugt 0,001 - 1,0 Gew.-% und besonders bevorzugt 0,01 - 0,5 Gew.-%, bezogen auf die gesamte Emulsion, enthalten.
In einer weiteren bevorzugten Ausführungsform enthalten die erfindungsgemäßen Öl-in-Wasser-Emulsionen als zusätzlichen kosmetischen Wirkstoff c) mindestens eine natürliche Betainverbindung. Diese Komponenten haben positive Effekte insbesondere bei der hautbefeuchtenden Behandlung. Erfindungsgemäß bevorzugte natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl.
Bevorzugte erfindungsgemäße Öl-in-Wasser-Emulsionen sind dadurch gekennzeichnet, dass sie mindestens eine natürliche Betainverbindung in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Emulsion, enthalten.

In einer weiteren bevorzugten Ausführungsform enthalten die erfindungsgemäßen Öl-in-Wasser-Emulsionen als zusätzlichen kosmetischen Wirkstoff c) mindestens ein Vitamin, Provitamin oder eine als Vitaminvorstufe bezeichnete Verbindung aus den Vitamingruppen A, B, C, E, H und K und den Estern der vorgenannten Substanzen. Diesen Komponenten werden positive Effekte insbesondere bei der Anti-Falten- und der Anti-Ageing-Behandlung, aber auch bei der hautbefeuchtenden, aufhellenden, sebumregulierenden und hautberuhigenden Behandlung zugeordnet.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester, wie Retinylpalmitat und Retinylacetat in Betracht. Die erfindungsgemäßen Emulsionen enthalten die mindestens eine Vitamin A-Komponente bevorzugt in einer Gesamtmenge von 0,05 - 1 Gew.-%, bezogen auf die gesamte Emulsion. Zur Vitamin B-Gruppe oder zu dem Vitamin B-Komplex gehören unter anderem
- Vitamin B₁, Trivialname Thiamin, chemische Bezeichnung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf die gesamte Zusammensetzung, eingesetzt.
- Vitamin B₂, Trivialname Riboflavin, chemische Bezeichnung 7,8-Dimethyl-10-(1-D-ribityl)-benzo[g]pteridin-2,4(3*H*,10*H*)-dion. Bevorzugt werden Riboflavin oder seine Derivate in einer Gesamtmenge von 0,05 bis 1 Gew.-%, bezogen auf die gesamte Zusammensetzung, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Zusammensetzungen bevorzugt in Mengen von 0,05 bis 1 Gew.-%, bezogen auf die gesamte Zusammensetzung, enthalten ist.
- Vitamin B₅ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder Panthenol auch Derivate des 2-Furanon mit der allgemeinen Strukturformel (VIT-I) eingesetzt werden.

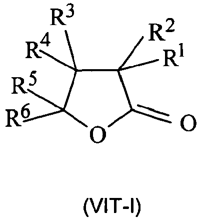

Bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (VIT-I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.
Die genannten Verbindungen des Vitamin B₅-Typs sowie die 2-Furanonderivate sind in den erfindungsgemäßen Zusammensetzungen bevorzugt in einer Gesamtmenge von 0,05 bis 5 Gew.-%, besonders bevorzugt 0,1 bis 3 Gew.-%, außerordentlich bevorzugt 0,5 bis 2 Gew.-%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxy-methyl-2-methylpyridin-3-ols versteht. Vitamin B₆ ist in den erfindungsgemäßen Zusammensetzungen bevorzugt in Mengen von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3a*S*,4*S*, 6a*R*)-2-Oxohexahydrothienol[3,4-*d*]-imidazol-4-valeriansäure. Biotin ist in den erfindungsgemäßen Zusammensetzungen bevorzugt in Mengen von 0,0001 bis 1,0 Gew.-%, insbesondere in Mengen von 0,001 bis 0,01 Gew.-% enthalten.
- Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Amino-benzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugte Zusammensetzungen sind dadurch gekennzeichnet, dass sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die Zusammensetzung, enthalten.
- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugte Zusammensetzungen sind dadurch gekennzeichnet, dass sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die Zusammensetzung, enthalten.

Zur Vitamin C-Gruppe zählen Vitamin C (Ascorbinsäure) und seine Derivate, insbesondere die Ester der Ascorbinsäure mit organischen und anorganischen Säuren und deren Salze, sowie die Acetale mit Glucose oder anderen Zuckern, insbesondere Ascorbylglucosid. Vitamin C und/oder mindestens eines seiner Derivate wird bevorzugt in einer Gesamtmenge von 0,1 bis 3 Gew.-%, bezogen auf die gesamte Zusammensetzung, eingesetzt. Die Verwendung der Derivate Ascorbylpalmitat, -stearat, -dipalmitat, -acetat, Mg-Ascorbylphosphat, Na-Ascorbylphosphat, Natrium- und Magnesiumascorbat, Dinatriumascorbylphosphat und -sulfat, Kaliumascorbyltocopherylphosphat, Chitosanascorbat oder Ascorbylglucosid kann bevorzugt sein. Die Verwendung mindestens eines Mitglieds der Vitamin C - Gruppe in Kombination mit Tocopherolen und/oder anderen Mitgliedern der Vitamin E - Gruppe kann ebenfalls bevorzugt sein.
Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in einer Gesamtmenge von 0,05 - 1 Gew.-%, bezogen auf die gesamte Zusammensetzung, enthalten.
Vitamin H ist eine andere Bezeichnung für Biotin oder Vitamin B₇ (siehe oben).
Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Vitamin K ist bevorzugt in Mengen von 0,0001 bis 1,0 Gew.-%, insbesondere 0,01 bis 0,5 Gew.-%, jeweils bezogen auf die gesamte Emulsion, enthalten. Vitamin A-palmitat (Retinylpalmitat), Panthenol, Pantolacton, Nicotinsäureamid, Pyridoxin, Pyridoxamin, Pyridoxal, Biotin, Ascorbylpalmitat, -acetat, Mg-Ascorbylphosphat, Na-Ascorbylphosphat, Natrium- und Magnesiumascorbat und die Tocopherolester, besonders Tocopherylacetat, sind erfindungsgemäß besonders bevorzugte Wirkstoffe c).

In einer weiteren bevorzugten Ausführungsform enthalten die erfindungsgemäßen Öl-in-Wasser-Emulsionen als zusätzlichen kosmetischen Wirkstoff c) mindestens ein Vitamin, Provitamin oder eine als Vitaminvorstufe bezeichnete Verbindung aus den Vitamingruppen A, B, C, E, H und K und den Estern der vorgenannten Substanzen in einer Gesamtmenge von 0,1 bis 5 Gew.-%, vorzugsweise von 0,25 bis 4 Gew.-% und insbesondere von 0,5 bis 2,5 Gew.-%, jeweils bezogen auf die gesamte Emulsion.
In einer weiteren bevorzugten Ausführungsform enthalten die erfindungsgemäßen Zusammensetzungen mindestens eine Substanz, die ausgewählt ist aus den Vitaminen, Provitaminen und Vitaminvorstufen der Gruppe B₁, B₂, B₃, B₆, B₇, B₉, B₁₃ und deren Estern und/oder Salzen und aus Pantolacton.

In einer weiteren bevorzugten Ausführungsform enthalten die erfindungsgemäßen Öl-in-Wasser-Emulsionen als zusätzlichen kosmetischen Wirkstoff c) mindestens eine α-Hydroxycarbonsäure, α-Ketocarbonsäure oder β-Hydroxycarbonsäure oder deren Ester-, Lacton- oder Salzform. Diesen Komponenten werden positive Effekte insbesondere bei der Anti-Falten- und der Anti-Ageing-Behandlung, aber auch bei der hautbefeuchtenden bzw. feuchtigkeitsspendenden, aufhellenden, sebumregulierenden und Anti-Akne-Behandlung zugeordnet. Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyocta-decansäure, Mandelsäure, 4-Hydroxymandelsäure, Äpfelsäure, Erythrarsäure, Threarsäure, Glucarsäure, Galactarsäure, Mannarsäure, 2-Hydroxy-2-methylbernsteinsäure, Gluconsäure, Gularsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren sind bevorzugt ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Besonders bevorzugte erfindungsgemäße Emulsionen sind dadurch gekennzeichnet, dass mindestens eine α-Hydroxy-carbonsäure, α-Ketocarbonsäure und/ oder β-Hydroxycarbonsäure und/oder mindestens ein Derivat hiervon in einer Gesamtmenge von 0,1 - 10 Gew.-%, bevorzugt 0,5 - 5 Gew.-%, jeweils bezogen auf die gesamte Emulsion, enthalten ist.
In einer weiteren bevorzugten Ausführungsform enthalten die erfindungsgemäßen Öl-in-Wasser-Emulsionen als zusätzlichen kosmetischen Wirkstoff c) mindestens ein Flavonoid und/oder mindestens einen Flavonoid-reichen Pflanzenextrakt. Diesen Komponenten werden positive Effekte insbesondere bei der Anti-Falten- und der Anti-Ageing-Behandlung, aber auch bei der hautbefeuchtenden bzw. feuchtigkeitsspendenden, aufhellenden, sebumregulierenden und Anti-Akne-Behandlung zugeordnet.

Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-gluco-pyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Diosmin (3',4',7-Trihydroxy-5-methoxyflavanon-7-rhamno-glucosid), Eriodictin und Apigenin-7-glucosid (4',5,7-Trihydroxyflavon-7-glucosid). Erfindungsgemäß außerordentlich bevorzugte Flavonoide sind α-Glucosylrutin, Naringin und Apigenin-7-glucosid.
Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin und Neohesperidindihydrochalkon.
Besonders bevorzugte erfindungsgemäße Emulsionen sind dadurch gekennzeichnet, dass mindestens ein Flavonoid und/oder mindestens einen Flavonoid-reicher Pflanzenextrakt in einer Gesamtmenge von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten Emulsion, enthalten ist.

In einer weiteren bevorzugten Ausführungsform enthalten die erfindungsgemäßen Öl-in-Wasser-Emulsionen als zusätzlichen kosmetischen Wirkstoff c) mindestens ein Isoflavonoid oder mindestens einen Isoflavonoid-reichen Pflanzenextrakt. Zu den Isoflavonoiden werden an dieser Stelle die Isoflavone und die Isoflavon-Glycoside gezählt. Diesen Komponenten werden positive Effekte insbesondere bei der Anti-Falten- und der Anti-Ageing-Behandlung zugeordnet.
Unter Isoflavonen sind im Sinne der vorliegenden Erfindung Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Als Isoflavone besonders bevorzugt sind Daidzein, Genistein, Glycitein und Formononetin.
In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin.
Erfindungsgemäß weiterhin bevorzugt ist es, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, insbesondere aus den Sojakeimen, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter der Produktbezeichnung Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoid-reicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse. Besonders bevorzugte erfindungsgemäße Emulsionen sind dadurch gekennzeichnet, dass als zusätzlicher kosmetischer Wirkstoff c) mindestens ein Isoflavonoid und/oder mindestens ein Isoflavonoid-reicher Pflanzenextrakt in einer Gesamtmenge von 0,00001 bis 1 Gew.-%, bevorzugt 0,0005 bis 0,5 Gew.-% und besonders bevorzugt 0,001 bis 0,1 Gew.-%, jeweils bezogen auf die Isoflavonoidaktivsubstanz in der gesamten Zusammensetzung, enthalten ist.

In einer weiteren bevorzugten Ausführungsform enthalten die erfindungsgemäßen Öl-in-Wasser-Emulsionen als zusätzlichen kosmetischen Wirkstoff c) mindestens ein Polyphenol und/oder mindestens einen Polyphenol-reichen Pflanzenextrakt. Diesen Komponenten werden positive Effekte insbesondere bei der Anti-Falten-, der Anti-Ageing-Behandlung und der sebumregulierenden Hautbehandlung zugeordnet.
Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Eichenrinde und Pinienrinde sowie anderen Baumrinden, Blättern von Grünem Tee (camellia sinensis) und Mate. Ebenfalls besonders bevorzugt sind die Tannine.
Ein besonders bevorzugter Polyphenol-reicher kosmetischer Wirkstoff ist das Handelsprodukt Sepivinol R, ein Extrakt aus Rotwein, erhältlich von der Firma Seppic. Ein weiterer besonders bevorzugter Polyphenol-reicher kosmetischer Wirkstoff ist das Handelsprodukt Crodarom Chardonnay, ein Extrakt aus den Kernen der Chardonnay-Traube, erhältlich von der Firma Croda. Besonders bevorzugte erfindungsgemäße Emulsionen sind dadurch gekennzeichnet, dass mindestens ein Polyphenol und/oder mindestens ein Polyphenol-reicher Pflanzenextrakt in einer Gesamtmenge von 0,001 bis 10 Gew.-%, bevorzugt 0,005 bis 5 Gew.-% und besonders bevorzugt 0,01 bis 3 Gew.-%, jeweils bezogen auf die Polyphenolaktivsubstanz in der gesamten Emulsion, enthalten ist.

In einer weiteren bevorzugten Ausführungsform enthalten die erfindungsgemäßen Öl-in-Wasser-Emulsionen als zusätzlichen kosmetischen Wirkstoff c) mindestens ein Ubichinon und/oder mindestens ein Ubichinol und/oder mindestens ein Derivat dieser Substanzen. Diesen Komponenten werden positive Effekte insbesondere bei der Anti-Falten- und der Anti-Ageing-Behandlung zugeordnet. Ubichinole sind die reduzierte Form der Ubichinone. Die erfindungsgemäß bevorzugten Ubichinone weisen die Formel (UBI-I) auf: 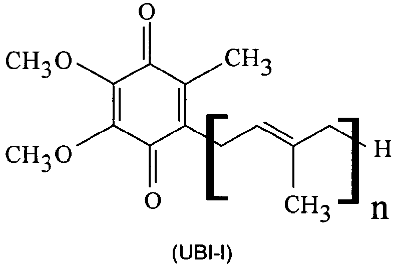 mit n = 6, 7, 8, 9 oder 10.
Besonders bevorzugt ist das Ubichinon der Formel (UBI-I) mit n = 10, auch bekannt als Coenzym Q10.
Besonders bevorzugte erfindungsgemäße Emulsionen sind dadurch gekennzeichnet, dass mindestens ein Ubichinon und/oder mindestens ein Ubichinol und/oder mindestens ein Derivat dieser Substanzen in einer Gesamtmenge von 0,0001 bis 1 Gew.-%, bevorzugt 0,001 bis 0,5 Gew.-% und besonders bevorzugt 0,005 bis 0,1 Gew.-%, jeweils bezogen auf die gesamte Emulsion, enthalten ist.

In einer weiteren bevorzugten Ausführungsform enthalten die erfindungsgemäßen Öl-in-Wasser-Emulsionen als zusätzlichen kosmetischen Wirkstoff c) Silymarin. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören. Diesen Komponenten werden positive Effekte insbesondere bei der hautberuhigenden Behandlung zugeordnet.
Besonders bevorzugte erfindungsgemäße Emulsionen sind dadurch gekennzeichnet, dass Silymarin in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Emulsion, enthalten ist.

In einer weiteren bevorzugten Ausführungsform enthalten die erfindungsgemäßen Öl-in-Wasser-Emulsionen als zusätzlichen kosmetischen Wirkstoff c) Ectoin. Dieser Komponente werden positive Effekte insbesondere bei der hautbefeuchtenden bzw. feuchtigkeitsspendenden Behandlung zugeordnet. Ectoin ist der Trivialname für 2-Methyl-1,4,5,6-tetrahydropyrimidin-4-carboxylat. Besonders bevorzugte erfindungsgemäße Emulsionen sind dadurch gekennzeichnet, dass Ectoin in Mengen von 0,0001 bis 1 Gew.-%, bevorzugt 0,001 bis 0,5 Gew.-% und besonders bevorzugt 0,005 bis 0,01 Gew.-%, jeweils bezogen auf die gesamte Zusammensetzung, enthalten ist.

In einer weiteren bevorzugten Ausführungsform enthalten die erfindungsgemäßen Öl-in- Wasser-Emulsionen als zusätzlichen kosmetischen Wirkstoff c) mindestens ein Repellent, das heißt, einen Wirkstoff zur Insektenabwehr.
Von den heute in Insektenabwehrmitteln ca. 15 häufig eingesetzten Wirkstoffen wird das N,N-Diethyl-3-methylbenzamid (DEET) als bestes Allround-Repellent bezeichnet. Es wirkt abwehrend gegen Stechmücken, Bremsen, Sandfliegen, Zecken, Stechfliegen, Milben, Flöhe und Wanzen, wobei die Wirkungsdauer - wie bei allen Repellent-Wirkstoffen - unterschiedlich lang gegenüber den verschiedenen Spezies ist. Handelsübliche DEET-Präparate beispielsweise sind ca. 6 bis 8 Stunden gegen Mücken wirksam, jedoch nur ca. 2 bis 4 Stunden gegen Zecken. Ein weiterer gebräuchlicher Repellent-Wirkstoff ist der 3-(N-n-Butyl-N-acetyl-amino)propionsäureethylester (auch als Repellent 3535 bezeichnet). Repellent 3535 ist gegen Stechmücken (Aedes aegypti, Anopheles albimanus), Tsetsefliegen (Glossinae) und Bremsen (Tabanidae) wirksam. Ferner gebräuchlich ist Dimethylphthalat (Palatinol M, DMP), das gegen Stechmücken (insbesondere Aedes- und Anopheles-Arten), Läuse, Zecken und Milben wirksam ist, allerdings vorwiegend in Kombination mit weiteren Repellent-Wirkstoffen eingesetzt wird.

In einer weiteren bevorzugten Ausführungsform enthalten die erfindungsgemäßen Öl-in-Wasser-Emulsionen als zusätzlichen kosmetischen Wirkstoff c) mindestens eine anorganische und/oder mindestens eine organische UV-Filtersubstanz.
Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme wieder abzugeben. Man unterscheidet UVA-Filter und UVB-Filter. Die UVA- und UVB-Filter können sowohl einzeln als auch in Mischungen eingesetzt werden. Der Einsatz von Filter-mischungen ist erfindungsgemäß bevorzugt. Die erfindungsgemäß bevorzugten organischen UV-Filter sind ausgewählt aus den Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Die Benzoxazol-Derivate liegen vorteilhaft in gelöster Form in den erfindungsgemäßen kosmetischen Zubereitungen vor. Es kann ggf. besonders bevorzugt sein, wenn die Benzoxazol-Derivate in pigmentärer, d. h. ungelöster Form - beispielsweise in Partikelgrößen von 10 nm bis zu 300 nm - vorliegen. Erfindungsgemäß besonders bevorzugte öllösliche UV-Filter sind 1-(4-tert.-Butylphenyl)-3-(4'-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 2-(4'-Diethylamino-2'-hydroxybenzoyl)-benzoesäurehexylester (auch: Aminobenzophenon, unter der Bezeichnung Uvinul A Plus bei der Firma BASF erhältlich), 4-Methoxybenzmalonsäuredi-2-ethylhexylester, an Polymere gebundene UV-Filter, z. B. das 3-(4-(2,2-Bis-Ethoxycarbonylvinyl)-phenoxy)propenyl)-methoxysiloxan/Dimethylsiloxan-Copolymer mit der INCI-Bezeichnung Dimethicodiethylbenzal malonate (CAS-Nr. 207574-74-1, Parsol^{®} SLX), Triazinderivate, wie z. B. 2,4-Bis-{[4-(2-Ethyl-hexyloxy)-2-hydroxy]-phenyl}-6-(4-methoxyphenyl)-1,3,5-triazin (INCl: Bis-Ethylhexyloxyphenol Methoxyphenyl Triazin, unter dem Namen Tinosorb S bei CIBA erhältlich), Dioctylbutylamidotriazon (INCl: Diethylhexyl Butamido Triazone, unter dem Namen Uvasorb^{®} HEB bei Sigma 3V erhältlich), 2,4,6-Trianilino-(p-carbo-2'-ethyl-1'-hexyloxy)-1,3,5-triazin (Ethylhexyl Triazone, Uvinul^{®} T 150), 2-[4,6-Bis(2,4-dimethylphenyl)-1,3,5-triazin-2-yl]-5-(octyloxy)phenol (CAS Nr.: 2725-22-6), 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma), die Benzotriazolderivate 2,2'-Methylen-bis-(6-(2H-benzotriazol-2-yl)-4-(1,1,3,3-tetramethylbutyl)-phenol) [Tinosorb M (Ciba)], 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'- Hydroxy-5'-octylphenyl)-benzotriazol (CAS-Nr. 003147-75-9), 2-(2'-Hydroxy-5'-methylphenyl) benzotriazol (CAS-Nr. 2440-22-4), 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane, 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin oder auch Aniso Triazin, erhältlich als Tinosorb^{®} S von CIBA), 2,4-Bis-{[4-(3-sulfonato)-2-hydroxy-propyloxy)-2-hydroxyl- phenyl}-6-(4-methoxyphenyl)-1,3,5-triazin-Natriumsalz, 2,4-Bis-{[4-(3-(2-propyloxy)-2-hydroxy-propyloxy)-2-hydroxy]-phenyl}-6-(4-methoxyphenyl)-1,3,5-triazin, 2,4-Bis-{[4-(2-ethyl-hexyloxy)-2-hydroxy]-phenyl}-6-[4-(2-methoxyethylcarboxyl)-phenylamino]-1,3,5-triazin, 2,4-Bis-{[4-(3-(2- propyloxy)-2-hydroxy-propyloxy)-2-hydroxy]-phenyl}-6-[4-(ethylcarboxyl)-phenylamino]-1,3,5-triazin, 2,4-Bis-{[4-(2-ethyl hexyloxy)-2-hydroxy]-phenyl}-6-(1-methyl-pyrrol-2-yl)-1,3,5-triazin, 2,4-Bis-{[4-tris(trimethylsiloxy-silylpropyloxy)-2-hydroxy]-phenyl}-6-(4-methoxyphenyl)-1,3,5-triazin, 2,4-Bis-{[4-(2-methylpropenyloxy)-2-hydroxy]-phenyl}-6-(4-methoxyphenyl)-1,3,5-triazin, 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin sowie Mischungen der genannten Komponenten.

Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, insbesondere die Sulfonsäure selbst mit der INCl Bezeichnung Phenylbenzimidazole Sulfonic Acid (CAS.-Nr. 27503-81-7), die beispielsweise unter dem Handelsnamen Eusolex 232 bei Merck oder unter Neo Heliopan Hydro bei Symrise erhältlich ist, und das Phenylen-1,4-bis-(2-benzimidazyl)-3,3'-5,5'-tetrasulfonsäure-bis- natriumsalz mit der INCI-Bezeichnung Disodium Phenyl Dibenzimidazol Tetrasulfonate (CAS-Nr.: 180898-37-7), das beispielsweise unter dem Handelsnamen Neo Heliopan AP bei Symrise erhältlich ist, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze mit der INCl-Bezeichnung Terephthalydene Dicampher Sulfonic Acid (CAS.-Nr.: 90457-82-2, als Mexoryl SX von der Firma Chimex erhältlich).

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.
Bevorzugte erfindungsgemäße Emulsionen sind dadurch gekennzeichnet, dass sie mindestens eine organische UV-Filtersubstanz in einer Gesamtmenge von 0,1 - 30 Gew.-%, bevorzugt 0,5 - 20 Gew.-%, besonders bevorzugt 1,0 - 10 Gew.-% und außerordentlich bevorzugt 2 oder 3 - 7 Gew.-%, jeweils bezogen auf die gesamte Zusammensetzung, enthalten.
Bevorzugte erfindungsgemäße Zusammensetzungen sind dadurch gekennzeichnet, dass sie mindestens eine anorganische UV-Filtersubstanz in einer Gesamtmenge von 0,1 - 15 Gew.-%, bevorzugt 0,5 - 10 Gew.-%, besonders bevorzugt 1 - 5 Gew.-% und außerordentlich bevorzugt 2 - 4 Gew.-%, jeweils bezogen auf die gesamte Zusammensetzung, enthalten.

In einer weiteren bevorzugten Ausführungsform enthalten die erfindungsgemäßen Öl-in- Wasser-Emulsionen als zusätzlichen kosmetischen Wirkstoff c) mindestens einen selbstbräunenden Wirkstoff. Erfindungsgemäß bevorzugte selbstbräunende Wirkstoffe sind ausgewählt aus Dihydroxyaceton, Erythrulose und 5,6-Dihydroxyindolin sowie Mischungen dieser Komponenten, insbesondere Mischungen von Dihydroxyaceton und Erythrulose.
Bevorzugte erfindungsgemäße Emulsionen sind dadurch gekennzeichnet, dass sie mindestens einen selbstbräunenden Wirkstoff in einer Gesamtmenge von 0,01 - 15 Gew.-%, bevorzugt 0,1 - 10 Gew.-%, besonders bevorzugt 1,0 - 5 Gew.-% und außerordentlich bevorzugt 2,0 - 4,0 Gew.-%, jeweils bezogen auf die gesamte Emulsion, enthalten.

In einer weiteren bevorzugten Ausführungsform enthalten die erfindungsgemäßen Öl-in-Wasser-Emulsionen als zusätzlichen kosmetischen Wirkstoff c) mindestens einen hautaufhellenden Wirkstoff. Erfindungsgemäß bevorzugte hautaufhellende Wirkstoffe sind ausgewählt aus Ascorbinsäure, den Estern der Ascorbinsäure mit Phosphorsäure und/ oder organischen C₂-C₂₀-Carbonsäuren sowie deren Alkali- und Erdalkalimetallsalzen, Kojisäure, Hydrochinon, Arbutin, Maulbeerbaumextrakt und Süßholzextrakt sowie Mischungen hiervon. Sowohl als Einzelsubstanz wie auch in Mischung bevorzugt sind die Ascorbinsäurederivate sowie Kojisäure bevorzugt. Besonders bevorzugt sind Natriumascorbylphosphat, Magnesiumascorbylphosphat, Ascorbylmonopalmitat, Ascorbyldipalmitat, Ascorbylmonostearat, Ascorbyldistearat, Ascorbylmonoethylhexanoat, Ascorbyldiethylhexanoat, Ascorbylmonooctanoat, Ascorbyldioctanoat, Ascorbylmonoisostearat und Ascorbyldiisostearat. Die erfindungsgemäß außerordentlich bevorzugten Ascorbinsäurederivate sind Natriumascorbylphosphat und Magnesiumascorbylphosphat.
Bevorzugte erfindungsgemäße Emulsionen sind dadurch gekennzeichnet, dass sie mindestens einen hautaufhellenden Wirkstoff in einer Gesamtmenge von 0,05 bis 5 Gew.%, bevorzugt 0,1 - 2 Gew-%, jeweils bezogen auf die gesamte Emulsion, enthalten.
In einer weiteren bevorzugten Ausführungsform enthalten die erfindungsgemäßen Öl-in-Wasser-Emulsionen als zusätzlichen kosmetischen Wirkstoff c) mindestens einen hautberuhigenden Wirkstoff. Erfindungsgemäß bevorzugte hautberuhigende Wirkstoffe sind ausgewählt aus Allantoin, α-Bisabolol, α-Liponsäure, Extrakten aus Centella asiatica, beispielsweise erhältlich unter der Bezeichnung Madecassicoside von DSM, Glycyrrethinsäure, die besonders bevorzugt in Liposomen verkapselt vorliegt und in dieser Form z. B. unter dem Handelsnamen Calmsphere von Soliance erhältlich ist, Mischungen aus Getreidewachsen, Extrakten aus Schibutter und Argania Spinosa-Öl mit der INCl-Bezeichnung "Spent grain wax and Butyrospermum Parkii (shea butter) extract and Argania Spinosa Kernel Oil", wie sie z. B. unter der Handelsbezeichnung Stimu-Tex AS von der Firma Pentapharm erhältlich sind, Extrakten aus Vanilla Tahitensis, wie sie z. B. unter der Handelsbezeichnung Vanirea (INCI : Vanilla Tahitensis Fruit Extract) von der Firma Solabia erhältlich sind, Alginhydrolysaten, wie sie z. B. unter der Handelsbezeichnung Phycosaccharide, insbesondere Phycosaccharide Al, von der Firma Codif erhältlich sind, Extrakten aus Bacopa Monniera, wie sie z. B. unter der Handelsbezeichnung Bacocalmine von der Firma Sederma erhältlich sind, Extrakten aus der Rooibos-Pflanze, wie sie z. B. unter dem Handelsnamen Rooibos Herbasec MPE von der Firma Cosmetochem erhältlich sind, Hefeextrakten, besonders bevorzugt das Handelsprodukt Drieline (INCI-Bezeichnung "Sorbitol, Yeast Extract"), erhältlich von der Firma Lanatech, den physiologisch verträglichen Salzen von Sterolsulfaten, wie sie z. B. unter der Handelsbezeichnung Phytocohesine (INCI: Sodium Beta-Sitosterylsulfate) von der Firma Vincience erhältlich sind, Aminodicarbonsäuren mit einer C-Kettenlänge von 3 - 6 Kohlenstoffatomen sowie deren physiologisch verträglichen Salzen, bevorzugt ausgewählt aus Aminomalonsäure, Aminobernsteinsäure (= Asparaginsäure), Aminoglutarsäure und Aminoadipinsäure sowie deren physiologisch verträglichen Salzen wie Kaliumaspartat und Magnesiumaspartat, sowie beliebigen Mischungen dieser Substanzen.
Weitere bevorzugte erfindungsgemäße Emulsionen sind dadurch gekennzeichnet, dass sie mindestens einen hautberuhigenden Wirkstoff in einer Gesamtmenge von 0,001 bis 5 Gew.-%, bevorzugt 0,01 bis 2 Gew.-% und besonders bevorzugt 0,1 bis 1 Gew.-%, jeweils bezogen auf die gesamte Emulsion, enthalten.
In einer weiteren bevorzugten Ausführungsform enthalten die erfindungsgemäßen Öl-in-Wasser-Emulsionen als zusätzlichen kosmetischen Wirkstoff c) mindestens einen feuchtigkeitsspendenden Wirkstoff. Erfindungsgemäß bevorzugte feuchtigkeitsspendende Wirkstoffe sind ausgewählt aus Desoxyzuckern, besonders bevorzugt Rhamnose und Fucose, Polysacchariden, die mindestens einen Desoxyzucker-Baustein enthalten, besonders bevorzugt aus den Handelsprodukten Fucogel^{®} (INCI-Bezeichnung Biosaccharide Gum-1) von Solabia, Rhamnosoft^{®} (INCI-Bezeichnung Biosaccharide Gum-2) von Solabia, Fucogenol^{®} (INCI-Bezeichnung Biosaccharide Gum-3) von Solabia und Glycofilm^{®} (INCI-Bezeichnung Biosaccharide Gum-4) von Solabia, weiterhin Mischungen der vorgenannten, mindestens einen Desoxyzucker-Baustein enthaltenden Polysaccharide, beispielsweise der Mischung aus Biosaccharide Gum-2 und Biosaccharide Gum-3, erhältlich als Handelsprodukt Elastinol plus^{®} von Solabia, weiterhin Harnstoff, N,N'-Bis(2-hydroxyethyl)harnstoff (z. B. erhältlich unter dem Handelsnamen Hydrovance), Betain (Me₃N⁺-CH₂-COO⁻), Glycosaminoglycanen, besonders bevorzugt Hyaluronsäure, Dextran, Dextransulfat, Chondroitin-4-sulfat und Chondroitin-6-sulfat, sowie beliebigen Mischungen dieser Substanzen.
Bevorzugte erfindungsgemäße Emulsionen sind dadurch gekennzeichnet, dass sie mindestens einen feuchtigkeitsspendenden Wirkstoff in einer Gesamtmenge von 0,001 bis 10 Gew.-%, bevorzugt 0,1 bis 5 Gew.-% und besonders bevorzugt 1 bis 3 Gew.-%, jeweils bezogen auf die gesamte Emulsion, enthalten.
In einer weiteren bevorzugten Ausführungsform enthalten die erfindungsgemäßen Öl-in-Wasser-Emulsionen als zusätzlichen kosmetischen Wirkstoff c) mindestens einen sebumregulierenden Wirkstoff. Erfindungsgemäß bevorzugte sebumregulierende Wirkstoffe sind ausgewählt aus Azelainsäure, Azelainsäurederivaten, insbesondere dem Azelainsäurederivat Potassium Azeloyl Diglycinate, das zum Beispiel als Handelsprodukt Azeloglicina von Sinerga erhältlich ist, Sebacinsäure, 10-Hydroxydecansäure, 1,10-Decandiol, Mischungen aus Sebacinsäure, 10-Hydroxydecansäure und 1,10-Decandiol, wie sie zum Beispiel als Handelsprodukt Acnacidol PG von Vincience erhältlich sind, Glycyrrhizin, das auch als Glycyrrhizinsäure oder Glycyrrhetinsäureglycosid bezeichnet wird und das 2-beta-Glucuronido-alpha-glucuronid der Glycyrrhetinsäure darstellt, sowie deren Salzen, Gerbsäure (tannic acid) und deren Salzen, Gallotanninen, Naringin, Mischungen aus Glycyrrhizin(salzen), Gerbsäure(salzen) und/oder Gallotanninen und Naringin, wie sie zum Beispiel als Handelsprodukt BiSCos Glynarin PF von der Firma Biesterfeld erhältlich sind, weiterhin aus Extrakten aus Spiraea Ulmaria, wie sie z. B. im Produkt Seboregul der Firma Silab enthalten sind, weiterhin aus wasser- und öllöslichen Extrakten aus Hamamelis, Klettenwurzel und Brennnessel, Zimtbaumextract (z. B. Sepicontrol^{®} A5 von der Firma Seppic), Chrysanthemenextrakt (z.B. Laricyl^{®} von Laboratoires Sérobiologiques), Hefeproteinhydrolysaten, wie sie z.B. in den Handelsprodukten der Asebiol^{®}-Serie von Laboratoires Sérobiologiques erhältlich sind, insbesondere Asebiol^{®} LS 2539 BT 2 (INCl: Aqua, Hydrolyzed Yeast Protein, Pyridoxine, Niacinamide, Glycerin, Panthenol, Allantoin, Biotin) und Asebiol^{®} LS 2539 BT (Aqua, Hydrolyzed Yeast Protein, Pyridoxine, Niacinamide, Glycerin, Panthenol, Propylene Glycol, Allantoin, Disodium Azelate, Biotin) und PEG-8 Isolauryl Thioether, wie es z. B. in dem Handelsprodukt "Antifettfaktor^{®} COS-218/2-A" von Cosmetochem enthalten ist (INCI: Aqua, Cetyl-PCA, PEG-8 Isolauryl Thioether, PCA, Cetyl Alcohol). Bevorzugte erfindungsgemäße Emulsionen sind dadurch gekennzeichnet, dass sie mindestens einen sebumregulierenden Wirkstoff in einer Gesamtmenge von 0,0001 bis 5 Gew.-%, bevorzugt 0,001 bis 2 Gew.-%, besonders bevorzugt 0,01 bis 1 Gew.-% und außerordentlich bevorzugt 0,1 - 0,5 Gew.-%, jeweils bezogen auf den Aktivsubstanzgehalt in der gesamten erfindungsgemäßen Emulsion, enthalten.

Weitere erfindungsgemäß bevorzugte Öl-in-Wasser-Emulsionen sind dadurch gekennzeichnet, dass die Öl- oder Fettphase mindestens einen Duftstoff umfasst.
Als Duftstoffkomponente können Parfüme, Parfümöle oder Parfümölbestandteile eingesetzt werden. Parfümöle bzw. Duftstoffe können erfindungsgemäß einzelne Riechstoffverbindungen, z. B. die synthetischen Produkte vom Typ der Ester, Ether, Aldehyde, Ketone, Alkohole und Kohlenwasserstoffe sein. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Zu den Ethern zählen beispielsweise Benzylethylether und Ambroxan , zu den Aldehyden z.B. die linearen Alkanale mit 8 - 18 C-Atomen, Citral, Citronellal, Citronellyloxy-acetaldehyd, Cyclamenaldehyd, Lilial und Bourgeonal, zu den Ketonen z.B. die Jonone, alpha-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen.
Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Laudanumöl sowie Orangenblütenöl, Neroliöl, Orangenschalenöl und Sandelholzöl.
Um wahrnehmbar zu sein, muss ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Aufgrund der unterschiedlichen Flüchtigkeit von Riechstoffen verändert sich der Geruch eines aus mehreren Riechstoffen zusammengesetzten Parfüms bzw. Duftstoffs während des Verdampfens, wobei man die Geruchseindrücke in "Kopfnote" (top note), "Herz- bzw. Mittelnote" (middle note bzw. body) sowie "Basisnote"
(end note bzw. dry out) unterteilt. Da die Geruchswahrnehmung zu einem großen Teil auch auf der Geruchsintensität beruht, besteht die Kopfnote eines Parfüms bzw. Duftstoffs nicht allein aus leichtflüchtigen Verbindungen, während die Basisnote zum größten Teil aus weniger flüchtigen, d.h. haftfesten Riechstoffen besteht. Bei der Komposition von Parfüms können leichter flüchtige Riechstoffe beispielsweise an bestimmte Fixative gebunden werden, wodurch ihr zu schnelles Verdampfen verhindert wird. Bei der nachfolgenden Einteilung der Riechstoffe in "leichter flüchtige" bzw. "haftfeste" Riechstoffe ist also über den Geruchseindruck und darüber, ob der entsprechende Riechstoff als Kopf- oder Herznote wahrgenommen wird, nichts ausgesagt.
Haftfeste Riechstoffe, die im Rahmen der vorliegenden Erfindung einsetzbar sind, sind beispielsweise die ätherischen Öle wie Angelikawurzelöl, Anisöl, Arnikablütenöl, Basilikumöl, Bayöl, Bergamotteöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl.
Aber auch die höhersiedenden bzw. festen Riechstoffe natürlichen oder synthetischen Ursprungs können im Rahmen der vorliegenden Erfindung als haftfeste Riechstoffe bzw. Riechstoffgemische, also Duftstoffe, eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylakohol, Benzylacetat, Benzylbenzoat, Benzylformiat, Benzylvalerianat, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphtholmethylether, Nerol, Nitrobenzol, n-Nonylaldehyd, Nonylalkohol, n-Octylaldehyd, p-Oxy-Acetophenon, Pentadekanolid, β-Phenylethylalkohol, Phenylacetaldehyd-Dimethylacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimatalkohol, Zimtsäure, Zimtsäureethylester, Zimtsäurebenzylester.
Zu den leichter flüchtigen Riechstoffen zählen insbesondere die niedriger siedenden Riechstoffe natürlichen oder synthetischen Ursprungs, die allein oder in Mischungen eingesetzt werden können. Beispiele für leichter flüchtige Riechstoffe sind Alkylisothiocyanate (Alkylsenföle), Butadion, Citral, Citronellal, Limonen, Linalool, Linaylacetat und -propionat, Menthol, Menthon, Methyl-n-heptenon, Phellandren, Phenylacetaldehyd und Terpinylacetat.

Besonders bevorzugte erfindungsgemäße kosmetische Emulsionen sind dadurch gekennzeichnet, dass mindestens eine Duftstoffkomponente in einer Gesamtmenge von 0,00001 bis 4 Gew.-%, bevorzugt 0,5 - 2 Gew.-%, besonders bevorzugt 1 - 1,5 Gew.-%, jeweils bezogen auf die Gesamtzusammensetzung, enthalten ist.
Weitere erfindungsgemäß bevorzugte Öl-in-Wasser-Emulsionen sind dadurch gekennzeichnet, dass sie in einem Behälter mit Kugelapplikator oder Roll-on-Applikator verpackt ist.
Ein weiterer Gegenstand der vorliegenden Erfindung ist die kosmetische, nicht-therapeutische Verwendung einer Öl-in-Wasser-Emulsion gemäß einem der Ansprüche 1 - 12 zur schweißhemmenden Behandlung der Haut, insbesondere der Achselhaut und/oder der Fußhaut. Ein weiterer Gegenstand der vorliegenden Erfindung ist die kosmetische, nicht-therapeutische Verwendung einer Öl-in-Wasser-Emulsion gemäß einem der Ansprüche 1 - 12 zur schweißhemmenden Behandlung der Haut, insbesondere der Achselhaut und/oder der Fußhaut, mit nicht-fettendem Hautgefühl.
Ein weiterer Gegenstand der vorliegenden Erfindung ist die kosmetische, nicht-therapeutische Verwendung einer Öl-in-Wasser-Emulsion gemäß einem der Ansprüche 1 - 12 zur schweißhemmenden Behandlung der Haut, insbesondere der Achselhaut und/oder der Fußhaut, mit beschleunigter Trocknung.
Ein weiterer Gegenstand der vorliegenden Erfindung ist ein kosmetisches, nicht-therapeutisches Verfahren zur schweißhemmenden Behandlung der Haut, insbesondere der Achselhaut und/oder der Fußhaut, das dadurch gekennzeichnet ist, dass eine Öl-in-Wasser-Emulsion gemäß einem der Ansprüche 1 - 12 in einer wirksamen Menge auf die Haut aufgetragen wird.

### Herstellung erfindungsgemäßer Emulsionen

Die erfindungsgemäßen Emulsionen können nach verschiedenen Herstellverfahren hergestellt werden. Erfindungsgemäß bevorzugte Herstellverfahren sind im folgenden dargestellt.

### Verfahren 1

Etwa ein Drittel der gesamten Wassermenge (Phase 1) und die Ölkomponente/n i) - iii) und der/die gegebenenfalls enthaltene/n Emulgator/en sowie ggf. weitere Öl- oder Fettkomponenten (Phase 2) werden getrennt voneinander auf eine Temperatur zwischen 70 - 80° C erwärmt. Dann wird Phase 1 langsam zu Phase 2 gegeben und alles 45 - 60 Minuten mit niedriger Drehzahl emulgiert bzw. auf kleiner Stufe homogenisiert. Anschließend wird der Ansatz auf 40 - 45 °C abgekühlt. Anschließend werden der kosmetische Rohstoff c) (sofern dieser ausreichend temperaturstabil ist) und 15 - 20 Gew.-% der gesamten Wassermenge auf 40 - 45 °C erwärmt, zum Ansatz gegeben und homogenisiert. Anschließend wird die noch verbleibende Portion der gesamten Wassermenge zusammen mit dem Polysaccharid und ggf. temperaturempfindlichen Wirkstoffen c) und Zusatzstoffen, beispielsweise Konservierungsmitteln, zum Ansatz gegeben, bei hoher Drehzahl homogenisiert und unter langsamem Rühren auf 25°C abgekühlt.
Die schweißhemmenden Wirkstoffe sind unter den genannten Herstellbedingungen nicht temperaturempfindlich und können bei 40 - 50°C eingearbeitet werden.
Die Dauer der einzelnen Homogenisierschritte beträgt 0,5 - 10 Minuten, bevorzugt 1 - 8 Minuten, besonders bevorzugt 2 - 5 Minuten.

### Verfahren 2

Etwa ein Drittel der gesamten Wassermenge (Phase 1) und die Ölkomponente/n i) - iii) und der/die gegebenenfalls enthaltene/n Emulgator/en sowie ggf. weitere Öl- oder Fettkomponenten (Phase 2) werden getrennt voneinander auf eine Temperatur zwischen 70 - 80° C erwärmt. Dann wird Phase 2 langsam zu Phase 1 gegeben und alles 45 - 60 Minuten mit niedriger Drehzahl emulgiert bzw. auf kleiner Stufe homogenisiert. Anschließend wird der Ansatz auf 40 - 45 °C abgekühlt. Anschließend werden der kosmetische Rohstoff c) (sofern dieser ausreichend temperaturstabil ist) und 15 - 20 Gew.-% der gesamten Wassermenge auf 40 - 45 °C erwärmt, zum Ansatz gegeben und homogenisiert. Anschließend wird die noch verbleibende Portion der gesamten Wassermenge zusammen mit dem Polysaccharid und ggf. temperaturempfindlichen Wirkstoffen c) und Zusatzstoffen, beispielsweise Konservierungsmitteln, zum Ansatz gegeben, bei hoher Drehzahl homogenisiert und unter langsamem Rühren auf 25°C abgekühlt.
Die schweißhemmenden Wirkstoffe sind unter den genannten Herstellbedingungen nicht temperaturempfindlich und können bei 40 - 50°C eingearbeitet werden.
Die Dauer der einzelnen Homogenisierschritte beträgt 0,5 - 10 Minuten, bevorzugt 1 - 8 Minuten, besonders bevorzugt 2 - 5 Minuten.

### Verfahren 3

Etwa 20 % der gesamten Wassermenge werden zusammen mit der/den Ölkomponente/n i) - iii) und den gegebenenfalls enthaltenden Emulgatoren auf eine Temperatur zwischen 70 - 80° C erwärmt und mit niedriger Drehzahl emulgiert bzw. auf kleiner Stufe homogenisiert.
Anschließend werden weitere 10 - 20 % der gesamten Wassermenge auf 70 - 80° C erwärmt, zugefügt, alles bei hoher Drehzahl homogenisiert und anschließend 0,5 - 2 Stunden lang emulgiert. Anschließend werden weitere 10 - 20 % der gesamten Wassermenge auf 70 - 80° C erwärmt, zugefügt und alles bei hoher Drehzahl homogenisiert. Der Ansatz wird auf 40 - 50°C abgekühlt. Eine Portion des kosmetischen Rohstoffs c) wird - sofern dieser Rohstoff bei dieser Temperatur stabil ist - mit weiteren 10 - 20 % der gesamten Wassermenge auf 40 - 50°C erwärmt, zum Ansatz zugegeben und alles bei hoher Drehzahl homogenisiert. Anschließend wird die restliche Portion des kosmetischen Rohstoffs c), sofern dieser Rohstoff bei dieser Temperatur stabil ist, mit weiteren 10 - 20 % der gesamten Wassermenge auf 40 - 50°C erwärmt, zum Ansatz gegeben und alles bei hoher Drehzahl homogenisiert.

Dann wird der Ansatz auf 30 - 35°C abgekühlt. Anschließend wird die noch verbleibende Portion der gesamten Wassermenge zugegeben, bei hoher Drehzahl homogenisiert und langsam unter Rühren abgekühlt.
Falls der kosmetische Rohstoff c) temperaturempfindlich ist, wird er nun erst zusammen mit dem Polysaccharid und ggf. weiteren (temperaturempfindlichen) Zusatzstoffen, beispielsweise Konservierungsmitteln, zum Ansatz gegeben, alles bei hoher Drehzahl homogenisiert und unter langsamem Rühren auf 25°C abgekühlt.
Die schweißhemmenden Wirkstoffe sind unter den genannten Herstellbedingungen nicht temperaturempfindlich und können bei allen Verfahren bei 40 - 50°C eingearbeitet werden.
Die Dauer der einzelnen Homogenisierschritte bei allen Verfahren beträgt 0,5 - 10 Minuten, bevorzugt 1 - 8 Minuten, besonders bevorzugt 2 - 5 Minuten.
Eine niedrige Scherrate liegt definitionsgemäß im Bereich von 1000 - 2500 Umdrehungen des Rührelementes pro Minute. Eine hohe Scherrate liegt definitionsgemäß im Bereich von 3000 - 6000 Umdrehungen des Rührelementes pro Minute.
Die nachfolgenden Beispiele sollen den Gegenstand der Erfindung näher erläutern, ohne ihn jedoch hierauf zu beschränken.

### Beispiel 1: erfindungsgemäße Antitranspirant-Emulsion (O/W) mit 1,9 Gew.-% Öl- und Fettphase

| **INGREDIENTS (EU-INCl)** | **Gew.-%-Anteil** | |
|---|---|---|
| AQUA | 81,0 | |
| ALUMINUM CHLOROHYDRATE | 13,0 | |
| STEARETH-2 | 2,5 | |
| STEARETH-21 | 1,5 | |
| PARFUM | 1,1 | Öl |
| PPG-15 STEARYL ETHER | 0,5 | Öl |
| BISABOLOL | 0,1 | Öl |
| ALUMINUM STARCH OCTENYLSUCCINATE | 0,1 | |
| BIS-PEG-18 METHYL ETHER DIMETHYL SILANE | 0,1 | Fett |
| TOCOPHERYL ACETATE | 0,1 | Öl |

Die Emulsion gemäß Beispiel 1 wies am ersten Tag nach der Herstellung eine Viskosität von 1800 mPas auf, gemessen mit einem Brookfield-Viskosimeter, Spindel RV 4, 20 s⁻¹, ohne Helipath, bei 20 °C Umgebungstemperatur und 20 °C Probentemperatur.

### Beispiel 2: erfindungsgemäße Antitranspirant-Emulsion (O/W) mit 1,5 Gew.-% Öl- und Fettphase

| **INGREDIENTS (EU-INCl)** | **Gew**.-**%**-**Anteil** | |
|---|---|---|
| AQUA | 68,3 | |
| ALUMINUM CHLOROHYDRATE | 26,0 | |
| STEARETH-2 | 2,4 | |
| STEARETH-21 | 1,5 | |
| PARFUM | 1,0 | Öl |
| PPG-15 STEARYL ETHER | 0,5 | Öl |
| ALLANTOIN | 0,1 | |
| ALUMINUM STARCH OCTENYLSUCCINATE | 0,1 | |
| ALOE BARBADENSIS | 0,1 | |

Die Emulsion gemäß Beispiel 2 wies am ersten Tag nach der Herstellung eine Viskosität von 2000 mPas auf, gemessen mit einem Brookfield-Viskosimeter, Spindel RV 4, 20 s⁻¹, ohne Helipath, bei 20 °C Umgebungstemperatur und 20 °C Probentemperatur.

### Beispiel 3: erfindungsgemäße Antitranspirant-Emulsion (O/W) mit 2,2 Gew.-% Öl- und Fettphase

| **INGREDIENTS (EU)** | **Gew.-%-Anteil** | |
|---|---|---|
| AQUA | 90,0 | |
| ALUMINUM ZIRCONIUM TETRACHLOROHYDREX GLY | 23,7 | |
| STEARETH-2 | 2,4 | |
| STEARETH-21 | 1,6 | |
| PARFUM | 1,2 | Öl |
| PPG-15 STEARYL ETHER | 0,5 | Öl |
| ALUMINUM STARCH OCTENYLSUCCINATE | 0,1 | |
| TOCOPHERYL ACETATE | 0,5 | Öl |

Die Emulsion gemäß Beispiel 3 wies am ersten Tag nach der Herstellung eine Viskosität von 2200 mPas auf, gemessen mit einem Brookfield-Viskosimeter, Spindel RV 4, 20 s⁻¹, ohne Helipath, bei 20 °C Umgebungstemperatur und 20 °C Probentemperatur.

### Beispiel 4: erfindungsgemäße Antitranspirant-Emulsion (O/W) mit 1,6 Gew.-% Öl- und Fettphase

| **INGREDIENTS (EU)** | **Gew.-%-Anteil** | |
|---|---|---|
| AQUA | 74,400000 | |
| ALUMINUM CHLOROHYDRATE | 20,000000 | |
| STEARETH-2 | 2,300000 | |
| STEARETH-21 | 1,500000 | |
| PARFUM | 1,000000 | Öl |
| PPG-15 STEARYL ETHER | 0,500000 | Öl |
| ALUMINUM STARCH OCTENYLSUCCINATE | 0,100000 | |
| ALLANTOIN | 0,100000 | |
| ISOPROPYL MYRISTATE | 0,100000 | Öl |

Die Emulsion gemäß Beispiel 4 wies am ersten Tag nach der Herstellung eine Viskosität von 1700 mPas auf, gemessen mit einem Brookfield-Viskosimeter, Spindel RV 4, 20 s⁻¹, ohne Helipath, bei 20 °C Umgebungstemperatur und 20 °C Probentemperatur.

### Beispiel 5: erfindungsgemäße Antitranspirant-Emulsion (O/W) mit 1,6 Gew.-% Öl- und Fettphase

| **INGREDIENTS (EU)** | **Gew.%-Anteil** | |
|---|---|---|
| AQUA | 74,400000 | |
| ALUMINUM CHLOROHYDRATE | 20,000000 | |
| STEARETH-2 | 2,300000 | |
| STEARETH-21 | 1,500000 | |
| PARFUM | 1,000000 | Öl |
| PPG-15 STEARYL ETHER | 0,500000 | Öl |
| DISTARCH PHOSPHATE | 0,100000 | |
| ALLANTOIN | 0,100000 | |
| ISOPROPYL MYRISTATE | 0,100000 | Öl |

Die Emulsion gemäß Beispiel 5 wies am ersten Tag nach der Herstellung eine Viskosität von 1800 mPas auf, gemessen mit einem Brookfield-Viskosimeter, Spindel RV 4, 20 s⁻¹, ohne Helipath, bei 20 °C Umgebungstemperatur und 20 °C Probentemperatur.

Die Emulsionen gemäß den Beispielen 1 - 5 wurden jeweils in eine Flasche mit einem Rollapplikator eingefüllt und waren so verkaufsfertig.

### Lagerstabilität

Alle erfindungsgemäßen Beispielemulsionen waren bei 40 °C Lagerung 12 Wochen lang stabil. Alle erfindungsgemäßen Beispielemulsionen waren bei 45 °C Lagerung 6 Wochen lang stabil. Bei der Lagerung bei 50 °C zeigte sich je nach Parfümöl eine leichte Aufrahmung zwischen der 3. und 4. Lagerwoche. Ohne die kritischen Parfümöle waren aber auch diese Emulsionen lagerstabil bei 50 °C.

### Untersuchungen zur Trockenzeit

Die erfindungsgemäße Beispielzusammensetzung 4 wurde mit zwei nicht-erfindungsgemäßen Vergleichsemulsionen folgender Zusammensetzung verglichen:
Vergleichsbeispiele V1 und V2: Nicht-erfindungsgemäße Antitranspirant-Emulsionen (O/W) ohne Polysaccharid (Mengen in Gew.-%)

| **INGREDIENTS (EU-INCl)** | **V1** | **V2** |
|---|---|---|
| AQUA | 74,500000 | 72,800000 |
| ALUMINUM CHLOROHYDRATE | 20,000000 | 20,000000 |
| STEARETH-2 | 2,300000 | 3.000000 |
| STEARETH-21 | 1,500000 | 1,000000 |
| PARFUM | 1,000000 | 1,000000 |
| PPG-15 STEARYL ETHER | 0,500000 | 2,000000 |
| ALUMINUM STARCH OCTENYLSUCCINATE | - | - |
| ALLANTOIN | 0,100000 | 0,100000 |
| ISOPROPYL MYRISTATE | 0,100000 | 0,100000 |

Die Probanden trugen jeweils eine definierte, identische Menge der Roll-on-Emulsionen von Beispiel 4, V1 oder V2 auf die Haut auf und bestimmten die Zeit (in Sekunden), nach der sie die Haut wieder als trocken empfanden.
Aus den Messwerten zur Trocknungszeit wurden die arithmetischen Mittelwerte gebildet. Diese sind in der folgenden Übersicht dargestellt.

| | **Beispiel 4** | **V1** | **V2** |
|---|---|---|---|
| Mittlere Trockenzeit [Sekunden] | 340 | 470 | 520 |

In einem weiteren Test mit 5 Testpersonen wurden verschiedene Polysaccharide miteinander verglichen. Außerdem wurden zwei stark ethanolhaltige Rezepturen mit getestet
Dazu wurden 0,15 g Produkt mit einer 1-ml-Spritze auf den Unterarm aufgetragen und anschließend mit dem Finger auf einer Strecke von 10 cm verteilt. Danach bestimmten die Probanden die Zeit (in Sekunden), nach der sie die Haut wieder als trocken empfanden. Die Werte sind in der nachfolgenden Tabelle zusammengestellt.

**Tabelle: Trockenzeit-Messungen**

| Rezeptur-Code | Testperson 1 [s] | Testperson 2 [s] | Testperson 3 [s] | Testperson 4 [s] | Testperson 5 [s] | mittlere Trockenzeit [Sekunden] | Bemerkung |
|---|---|---|---|---|---|---|---|
| 43/01 | 605 | 230 | 220 | 600 | 240 | 379 | weißelt nur ganz leicht beim Auftragen |
| 43/02 | 625 | 245 | 245 | 350 | 180 | 329 | weißelt beim Auftragen, pudrig |
| 43/03 | 675 | 200 | 320 | 540 | 240 | 395 | |
| 43/04 | (945) | 180 | 300 | 615 | 150 | (438) 326 (ohne Test 1) | weißelt beim Auftragen |
| 43/05 | 390 | 350 | 310 | 450 | 230 | 346 | weißelt leicht beim Auftragen |
| 43/06 | 350 | 205 | 350 | 505 | 240 | 330 | sehr angenehmes Hautgefühl |
| 97/01 | 645 | 150 | 580 | 610 | 365 | 470 | starker weißer Rückstand |
| 11/01 | 860 | 165 | 530 | 705 | 380 | 528 | weißelt beim Auftragen |
| 32/07 | 125 | 205 | 210 | 120 | 300 | 193 | |
| 16/02 | 140 | 220 | 100 | 140 | 170 | 148 | |

Durch die Verwendung von 0,1 - 0,3 Gew.-% Polysaccharid wurde die "gefühlte" Trockenzeit im Vergleich zu den Polysaccharid-freien Rezepturen 97/01 und 11/01 sehr deutlich reduziert.

Die Rezepturen 32/07 und 16/02 sind schnell trocknend, was für den Fachmann auf den hohen Ethanolgehalt zurückzuführen war. Ein Einfluss des Polysaccharidgehaltes auf die "gefühlte" Trockenzeit war, hiervon ausgehend, für den Fachmann nicht zu erwarten.

Verwendete Rezepturen (Mengenangaben in Gew.-%)

| | Code 43/01 | Code 43/02 | Code 43/03 | Code 43/04 | Code 43/05 | Code 43/06 | Code 97/01 | Code 11/01 | Code 32/07 | Code 16/02 |
|---|---|---|---|---|---|---|---|---|---|---|
| AQUA | 75,45 | 75,25 | 75,45 | 75,25 | 74,35 | 74,35 | 74,30 | 72,70 | 44,5441 | 44,5445 |
| STEARETH-2 | 2,40 | 2,40 | 2,40 | 2,40 | 2,40 | 2,40 | 2,40 | 3,00 | - | - |
| PPG-15 STEARYL ETHER | 0,50 | 0,50 | 0,50 | 0,50 | 0,50 | 0,50 | 0,50 | 2,00 | - | - |
| STEARETH-21 | 1,50 | 1,50 | 1,50 | 1,50 | 1,50 | 1,50 | 1,50 | 1,00 | - | - |
| ALUMINUM CHLOROHYDRATE | 20,00 | 20,00 | 20,00 | 20,00 | 20,00 | 20,00 | 20,00 | 20,00 | 20,00 | 17,00 |
| ALLANTOIN | - | - | - | - | - | - | 0,10 | 0,10 | - | - |
| ISOPROPYL MYRISTATE | - | - | - | - | - | - | 0,10 | 0,10 | - | - |
| PERFUME | - | - | - | - | 1,10 | 1,10 | 1,10 | 1,10 | - | - |
| NaOH | - | - | - | - | - | - | - | - | 0,0009 | 0,0005 |
| ETHANOL 96% | - | - | - | - | - | - | - | - | 30,00 | 33,00 |
| CETEARETH-12 | - | - | - | - | - | - | - | - | 2,00 | 2,00 |
| CETEARETH-30 | - | - | - | - | - | - | - | - | 2,00 | 2,00 |
| BIS-PEG-18 METHYL ETHER DIMETHYL SILANE | - | - | - | - | - | - | - | - | 1,00 | 1,00 |
| TOCOPHERYL ACETATE | 0,05 | 0,05 | 0,05 | 0,05 | 0,05 | 0,05 | - | - | 0,05 | 0,05 |
| ALOE POWDER | - | - | - | - | - | - | - | - | 0,005 | 0,005 |
| HYDROXYETHYLCELLULOSE | - | - | - | - | - | - | - | - | 0,40 | 0,40 |
| DISTARCH PHOSPHATE | 0,10 | 0,30 | - | - | 0,10 | - | - | - | - | - |
| ALUMINUM STARCH OCTENYLSUCCINATE | - | - | 0,10 | 0,30 | - | 0,10 | - | - | - | - |

## Patentansprüche

1. Kosmetische Öl-in-Wasser-Emulsion, die keine Mikroemulsion darstellt, enthaltend
a) 0,5 - 6,5 Gew.-% Öl- oder Fettphase, umfassend mindestens eine bei 20 °C flüssige Ölkomponente, ausgewählt aus
i) linearen und verzweigten gesättigten ein- oder mehrwertigen C₃ - C₃₀-Alkanolen, die mit mindestens einer Propylenoxid-Einheit pro Molekül verethert sind,
ii) Propylenglycolmonoestern von verzweigten gesättigten C₆ - C₃₀-Alkancarbonsäuren,
iii) verzweigten gesättigten C₁₀ - C₃₀-Alkanolen,
b) mindestens 60 Gew.-% Wasser,
c) 0,00001 - 38 Gew.-% mindestens eines kosmetischen Wirkstoffes, ausgewählt aus
• schweißhemmenden Wirkstoffen,
• desodorierenden Wirkstoffen,
• Monomeren, Oligomeren und Polymeren von Aminosäuren, N-C₂-C₂₄-Acylaminosäuren, den Estern und/oder den physiologisch verträglichen Salzen dieser Substanzen,
• DNA- oder RNA-Oligonucleotiden,
• natürlichen Betainverbindungen,
• Vitaminen, Provitaminen und Vitaminvorstufen der Gruppen A, B, C, E, H und K und den Estern der vorgenannten Substanzen,
• α-Hydroxycarbonsäuren, α-Ketocarbonsäuren, β-Hydroxycarbonsäuren und deren Ester-, Lacton- oder Salzform,
• Flavonoiden und Flavonoid-reichen Pflanzenextrakten,
• Isoflavonoiden und Isoflavonoid-reichen Pflanzenextrakten,
• Polyphenolen und Polyphenol-reichen Pflanzenextrakten,
• Ubichinon und Ubichinol sowie deren Derivaten,
• Silymarin,
• Ectoin,
• Repellentien,
• anorganischen und organischen UV-Filtersubstanzen,
• selbstbräunenden Wirkstoffen,
• hautaufhellenden Wirkstoffen,
• hautberuhigenden Wirkstoffen,
• feuchtigkeitsspendenden Wirkstoffen,
• sebumregulierenden Wirkstoffen,
wobei mindestens ein schweißhemmender Wirkstoff enthalten ist,
d) mindestens ein Polysaccharid in einer Gesamtmenge von 0,01 - 0,2 Gew.-%,
e) mindestens einen nichtionischen Emulgator mit einem HLB-Wert im Bereich von 3 - 6 in einer Gesamtmenge von 1,8 - 3 Gew.%,
f) mindestens einen nichtionischen Emulgator mit einem HLB-Wert im Bereich von 12 - 18, ausgewählt aus Steareth, Ceteth, Myristeth, Laureth, Trideceth, Arachideth und Beheneth mit jeweils 7 - 40 Ethylenoxid-Einheiten pro Molekül, in einer Gesamtmenge von 1 - 2 Gew.-%,
wobei alle Mengenangaben auf das Gesamtgewicht der Emulsion bezogen sind und das Gewichtsverhältnis von nichtionischen Emulgatoren mit einem HLB-Wert im Bereich von 3 - 6 und nichtionischen Emulgatoren mit einem HLB-Wert im Bereich von 12 - 18 von 0,9 bis 3, bevorzugt 1,3 -1,9 beträgt und die Emulgatoren definitionsgemäß nicht zur Öl- oder Fettphase zählen, **gekennzeichnet durch** eine Viskosität im Bereich von 1000 - 5000 mPas, gemessen 1 Tag nach Herstellung mit einem Brookfield-Viskosimeter, Spindel RV 4, 20 s⁻¹, ohne Helipath, bei 20 °C Umgebungstemperatur und 20 °C Probentemperatur.

2. Kosmetische Öl-in-Wasser-Emulsion gemäß Anspruch 1, **dadurch gekennzeichnet, dass** das mindestens eine Polysaccharid ausgewählt ist aus anionischen und nichtionischen Polysacchariden sowie Mischungen hiervon.

3. Kosmetische Öl-in-Wasser-Emulsion gemäß Anspruch 1 oder 2, **dadurch gekennzeichnet, dass** das anionische Polysaccharid ausgewählt ist aus Aluminiumstärkeoctenylsuccinat, Natriumstärkeoctenylsuccinat, Calciumstärkeoctenylsuccinat, Distärkephosphaten, Hydroxyethylstärkephosphaten, Hydroxypropylstärkephosphaten, Natriumcarboxymethylstärken, Natriumstärkeglycolat sowie Mischungen hiervon.

4. Kosmetische Öl-in-Wasser-Emulsion gemäß einem der vorstehenden Ansprüche, **dadurch gekennzeichnet, dass** die Ölkomponente i) ausgewählt ist aus Anlagerungsprodukten von mindestens 6 Propylenoxid-Einheiten pro Molekül an ein- oder mehrwertige C₃₋₃₀-Alkanole, insbesondere an Butanol, Butandiol, Myristylalkohol und Stearylalkohol.

5. Kosmetische Öl-in-Wasser-Emulsion gemäß einem der vorstehenden Ansprüche, **dadurch gekennzeichnet, dass** die Ölkomponente i) ausgewählt ist aus PPG-13-Butylether, PPG-14-Butylether, PPG-9-Butylether, PPG-10-Butandiol und PPG-15-Stearylether sowie Mischungen hiervon.

6. Kosmetische Öl-in-Wasser-Emulsion gemäß einem der vorstehenden Ansprüche, **dadurch gekennzeichnet, dass** die Ölkomponente ii) ausgewählt ist aus Propylenglycolmonoisostearat, Propylenglycolmonoisopalmitat, Propylenglycolmonoisobehenat, Propylenglycolmonoisoarachinat, Propylenglycolmonoisomyristat, Propylenglycolmonoisocaprat, Propylenglycolmonoisocaprinat und Propylenglycolmonoisocaprylat sowie Mischungen hiervon.

7. Kosmetische Öl-in-Wasser-Emulsion gemäß einem der vorstehenden Ansprüche, **dadurch gekennzeichnet, dass** die Ölkomponente iii) ausgewählt ist aus Isostearylalkohol, Isocetylalkohol, Isomyristylalkohol, Isotridecylalkohol, Isoarachidylalkohol, Isobehenylalkohol, Isocaprylalkohol, Isocaprinylalkohol, Isocaprylylalkohol, sowie Mischungen hiervon.

8. Kosmetische Öl-in-Wasser-Emulsion gemäß einem der vorstehenden Ansprüche, **dadurch gekennzeichnet, dass** der mindestens eine nichtionische Emulgator mit einem HLB-Wert im Bereich von 3 - 6 ausgewählt ist aus Steareth, Ceteth, Myristeth, Laureth, Trideceth, Arachideth und Beheneth mit jeweils 1 - 4 Ethylenoxid-Einheiten pro Molekül.

9. Kosmetische Öl-in-Wasser-Emulsion gemäß einem der vorstehenden Ansprüche, **dadurch gekennzeichnet, dass** als nichtionischer Emulgator mit einem HLB-Wert im Bereich von 3 - 6 Steareth-2 und gleichzeitig als nichtionischer Emulgator mit einem HLB-Wert im Bereich von 12 - 18 Steareth-21 enthalten ist.

10. Kosmetische Öl-in-Wasser-Emulsion gemäß einem der vorstehenden Ansprüche, **dadurch gekennzeichnet, dass** Steareth-2, Steareth-21 und PPG-15-Stearylether enthalten sind.

11. Kosmetische Öl-in-Wasser-Emulsion gemäß Anspruch 10, **dadurch gekennzeichnet, dass** mindestens ein Polysaccharid, ausgewählt aus Aluminiumstärkeoctenylsuccinat und Distärkephosphaten, enthalten ist.

12. Kosmetische Öl-in-Wasser-Emulsion gemäß einem der vorstehenden Ansprüche, **dadurch gekennzeichnet, dass** insgesamt maximal 3 Gew.-%, bevorzugt maximal 1 Gew.-% und besonders bevorzugt 0 Gew.-%, jeweils bezogen auf das Gewicht der gesamten Emulsion, an einwertigen C₁ - C₃-Alkanolen, wie Ethanol oder Isopropanol, enthalten ist.

13. Kosmetische, nicht-therapeutische Verwendung einer kosmetischen Öl-in-Wasser-Emulsion gemäß einem der Ansprüche 1 - 12 zur schweißhemmenden Behandlung der Haut, insbesondere der Achselhaut und/oder der Fußhaut, mit nicht-fettendem Hautgefühl und/oder mit beschleunigter Trocknung.

14. Kosmetisches, nicht-therapeutisches Verfahren zur schweißhemmenden Behandlung der Haut, insbesondere der Achselhaut und/oder der Fußhaut, **dadurch gekennzeichnet, dass** eine Öl-in-Wasser-Emulsion gemäß einem der Ansprüche 1 - 12 in einer wirksamen Menge auf die Haut aufgetragen wird.

## Claims

1. A cosmetic oil-in-water emulsion that does not represent a microemulsion, containing
a) 0.5 to 6.5 wt% oil phase or fat phase, encompassing at least one oil component that is liquid at 20°C, selected from
i) linear and branched saturated mono- or polyvalent C₃ to C₃₀ alkanols that are etherified with at least one propylene oxide unit per molecule,
ii) propylene glycol monoesters of branched saturated C₆ to C₃₀ alkanecarboxylic acids,
iii) branched saturated C₁₀ to C₃₀ alkanols,
b) at least 60 wt% water,
c) 0.00001 to 38 wt% of at least one cosmetic active substance selected from
- perspiration-inhibiting active substances,
- deodorizing active substances,
- monomers, oligomers, and polymers of amino acids, N-C₂-C₂₄ acylamino acids, the esters and/or the physiologically compatible salts of said substances,
- DNA or RNA oligonucleotides,
- natural betaine compounds,
- vitamins, provitamins, and vitamin precursors of groups A, B, C, E, H, and K, and the esters of the aforesaid substances,
- α-hydroxycarboxylic acids, α-ketocarboxylic acids, β-hydroxycarboxylic acids, and their ester, lactone, or salt form,
- flavonoids and flavonoid-rich plant extracts,
- isoflavonoids and isoflavonoid-rich plant extracts,
- polyphenols and polyphenol-rich plant extracts,
- ubiquinone and ubiquinol, and derivatives thereof,
- silymarin,
- ectoin,
- repellents,
- inorganic and organic UV-filtering substances,
- self-tanning active substances,
- skin-lightening active substances,
- skin-calming active substances,
- moisture-donating active substances,
- sebum-regulating active substances,
wherein at least one perspiration-inhibiting active substance is contained,
d) at least one polysaccharide in a total quantity from 0.01 to 0.2 wt%,
e) at least one nonionic emulsifier having an HLB value in the range from 3 to 6 in a total quantity from 1.8 to 3 wt%,
f) at least one nonionic emulsifier having an HLB value in the range from 12 to 18, selected from steareth, ceteth, myristeth, laureth, trideceth, arachideth, and beheneth, having respectively 7 to 40 ethylene oxide units per molecule, in a total quantity from 1 to 2 wt%,
all the quantitative indications being based on the total weight of the emulsion,
and wherein the weight ratio of nonionic emulsifiers having an HLB value in the range from 3 to 6, and nonionic emulsifiers having an HLB value in the range from 12 to 18, is from 0.9 to 3, preferably 1.3 to 1.9,
and wherein, by definition, the emulsifiers do not belong to the oil phase or fat phase,
**characterized by** a viscosity in the range from 1000 to 5000 mPas, measured one day after manufacture with a Brookfield viscosimeter, RV 4 spindle, 20 s⁻¹, without Helipath, at 20°C ambient temperature and 20°C sample temperature.

2. The cosmetic oil-in-water emulsion according to Claim 1, wherein the at least one polysaccharide is selected from anionic and nonionic polysaccharides as well as mixtures thereof.

3. The cosmetic oil-in-water emulsion according to Claim 1 or 2, wherein the anionic polysaccharide is selected from aluminum starch octenylsuccinate, sodium starch octenylsuccinate, calcium starch octenylsuccinate, distarch phosphates, hydroxyethyl starch phosphates, hydroxypropyl starch phosphates, sodium carboxymethyl starches, sodium starch glycolate, and mixtures thereof.

4. The cosmetic oil-in-water emulsion according to one of the preceding claims, wherein the oil component i) is selected from addition products of at least six propylene oxide units per molecule to mono- or polyvalent C₃₋₃₀ alkanols, in particular to butanol, butanediol, myristyl alcohol, and stearyl alcohol.

5. The cosmetic oil-in-water emulsion according to one of the preceding claims, wherein the oil component i) is selected from PPG-13 butyl ether, PPG-14 butyl ether, PPG-9 butyl ether, PPG-10 butanediol, and PPG-15 stearyl ether, and mixtures thereof.

6. The cosmetic oil-in-water emulsion according to one of the preceding claims, wherein the oil component ii) is selected from propylene glycol monoisostearate, propylene glycol monoisopalmitate, propylene glycol monoisobehenate, propylene glycol monoisoarachidate, propylene glycol monoisomyristate, propylene glycol monoisocaprate, propylene glycol monoisocaprinate, and propylene glycol monoisocaprylate, and mixtures thereof.

7. The cosmetic oil-in-water emulsion according to one of the preceding claims, wherein the oil component iii) is selected from isostearyl alcohol, isocetyl alcohol, isomyristyl alcohol, isotridecyl alcohol, isoarachidyl alcohol, isobehenyl alcohol, isocapryl alcohol, isocaprinyl alcohol, isocaprylyl alcohol, and mixtures thereof.

8. The cosmetic oil-in-water emulsion according to one of the preceding claims, wherein the at least one nonionic emulsifier having an HLB value in the range from 3 to 6 is selected from steareth, ceteth, myristeth, laureth, trideceth, arachideth, and beheneth, having respectively 1 to 4 ethylene oxide units per molecule.

9. The cosmetic oil-in-water emulsion according to one of the preceding claims, wherein steareth-2 is contained as a nonionic emulsifier having an HLB value in the range from 3 to 6, and steareth-21 is simultaneously contained as a nonionic emulsifier having an HLB value in the range from 12 to 18.

10. The cosmetic oil-in-water emulsion according to one of the preceding claims, wherein steareth-2, steareth-21, and PPG-15 stearyl ether are contained.

11. The cosmetic oil-in-water emulsion according to Claim 10, wherein at least one polysaccharide, selected from aluminum starch octenylsuccinate and distarch phosphates, is contained.

12. The cosmetic oil-in-water emulsion according to one of the preceding claims, wherein a total of at most 3 wt%, preferably at most 1 wt%, and particularly preferably 0 wt%, based in each case on the weight of the entire emulsion, of monovalent C₁ to C₃ alkanols, such as ethanol or isopropanol, is contained.

13. Cosmetic, non-therapeutic use of an oil-in-water emulsion according to one of Claims 1 to 12 for perspiration-inhibiting treatment of the skin, in particular the armpit skin and/or foot skin, with a non-greasy skin feel and/or with accelerated drying.

14. Cosmetic, non-therapeutic method for perspiration-inhibiting treatment of the skin, in particular the armpit skin and/or foot skin, wherein an oil-in-water emulsion according to one of Claims 1 to 12 is applied in an effective quantity onto the skin.

## Revendications

1. Emulsion cosmétique huile-dans-eau, qui n'est pas une microémulsion, contenant
a) de 0,5 à 6,5% en poids de phase huileuse ou graisseuse comprenant au moins un composant d'huile liquide à 20°C, choisi parmi
i) les alcanols en C₃ à C₃₀ monovalents ou polyvalents saturés linéaires ou ramifiés qui sont éthérifiés avec au moins une unité d'oxyde de propylène par molécule,
ii) les monoesters de propylène glycol et d'acides alcane-carboxyliques en C₆ à C₃₀ saturés et ramifiés,
iii) les alcanols en C₁₀ à C₃₀ saturés et ramifiés,
b) au moins 60% en poids d'eau,
c) 0,00001 à 38% en poids d'au moins un agent actif cosmétique choisi parmi
• les agents actifs anti-transpirants,
• les agents désodorisants,
• les monomères, les oligomères et les polymères d'acides aminés, les acides aminés N-acyles en C₂-C₂₄, les esters et/ou les sels physiologiquement acceptables de ces substances,
• les oligonucléotides d'ADN ou d'ARN,
• les composés naturels de la bétaïne,
• les vitamines, les provitamines et les précurseurs de vitamines des groupes A, B, C, E, H et K et les esters des substances susmentionnées,
• les acides α-hydroxy-carboxyliques, les acides α-céto-carboxyliques, les acides β-hydroxy-carboxyliques et leur forme ester, lactone ou sel,
• les flavonoïdes et les extraits de plantes riches en flavonoïdes,
• les isoflavonoïdes et les extraits de plantes riches en isoflavonoïdes,
• les polyphénols et les extraits de plantes riches en polyphénols,
• l'ubiquinone et l'ubiquinol et leurs dérivés,
• la silymarine,
• l'ectoïne,
• les répulsifs,
• les substances organiques et minérales filtrant les UV,
• les agents auto-bronzants,
• les agents d'éclaircissement de la peau,
• les agents apaisants de la peau,
• les agents hydratants,
• les agents de régulation du sébum,
avec au moins un agent anti-transpirant,
d) au moins un polysaccharide dans une quantité totale de 0,01 à 0,2% en poids,
e) au moins un émulsifiant non ionique ayant une valeur HLB dans la gamme de 3 à 6 dans une quantité totale de 1,8 à 3% en poids,
f) au moins un émulsifiant non ionique ayant une valeur HLB dans la gamme de 12 à 18, choisi parmi stéareth, ceteth, myristeth, laureth, trideceth, arachideth et beheneth comportant chacun 7 à 40 unités d'oxyde d'éthylène par molécule, dans une quantité totale de 1 à 2% en poids,
toutes les quantités étant basées sur le poids total de l'émulsion et le rapport en poids entre les émulsions non ioniques ayant une valeur HLB dans la gamme de 3 à 6 et les émulsions non ioniques ayant une valeur HLB dans la gamme de 12 à 18 étant de 0,9 à 3, de préférence 1,3 à 1,9, et les émulsions n'appartenant pas par définition à la phase huileuse ou graisseuse, **caractérisée par** une viscosité dans la gamme de 1000 à 5000 mPas, mesurée 1 jour après la production avec un viscosimètre Brookfield, broche RV 4, 20 s⁻¹, sans Helipath, à une température ambiante de 20°C et une température d'échantillon de 20°C.

2. Emulsion cosmétique huile-dans-eau selon la revendication 1, **caractérisée en ce que** l'au moins un polysaccharide est choisi parmi les polysaccharides anioniques et non ioniques et leurs mélanges.

3. Emulsion cosmétique huile-dans-eau selon la revendication 1 ou 2, dans laquelle le polysaccharide anionique est choisi parmi l'octénylsuccinate d'amidon d'aluminium, l'octénylsuccinate d'amidon de sodium, l'octénylsuccinate d'amidon de calcium, les phosphates de diamidon, les phosphates d'hydroxyéthylamidon, les phosphates d'hydroxypropylamidon, les carboxyméthylamidon de sodium, le glycolate d'amidon de sodium et leurs mélanges.

4. Emulsion cosmétique huile-dans-eau selon l'une des revendications précédentes, **caractérisée en ce que** la composante huileuse i) est choisie parmi les produits d'addition d'au moins 6 unités d'oxyde de propylène par molécule à des alcanols en C₃ à C₃₀ monovalents ou polyvalents, en particulier au butanol , au butanediol, à l'alcool myristique et à l'alcool stéarique.

5. Emulsion cosmétique huile-dans-eau selon l'une des revendications précédentes, **caractérisée en ce que** la composante huileuse est i) choisie parmi l'éther de PPG-13-butyle, l'éther de PPG-14-butyle, l'éther de PPG-9-butyle, le PPG-10-butanediol et l'éther de PPG-15-stéaryle et leurs mélanges.

6. Emulsion cosmétique huile-dans-eau selon l'une des revendications précédentes, **caractérisée en ce que** la composante huileuse ii) est choisie parmi le monoisostéarate de propylène glycol, le monoisopalmitate de propylène glycol, le monoisobéhénate de propylène glycol, le monoisoarachinate de propylène glycol, le monoisomyristate de propylène glycol, le monoisocaprate de propylène glycol, le monoisocaprinate de propylène glycol et le monoisocaprylate de propylène glycol et leurs mélanges.

7. Emulsion cosmétique huile-dans-eau selon l'une des revendications précédentes, **caractérisée en ce que** la composante huileuse iii) est choisie parmi l'alcool isostéarique, l'alcool isocétylique, l'alcool isomyristique, l'alcool isotridécylique, l'alcool isoarachidylique, l'alcool isobéhénylique, l'alcool isocaprylique, l'alcool isocaprinylique, l'alcool isocaprylylique et leurs mélanges.

8. Emulsion cosmétique huile-dans-eau selon l'une des revendications précédentes, **caractérisée en ce que** l'au moins un émulsifiant non ionique ayant une valeur HLB dans la gamme de 3 à 6 est choisi parmi Stéareth, Ceteth, Myristeth, Laureth, Trideceth, Arachideth, Beheneth ayant chacun 1 à 4 unités d'oxyde d'éthylène par molécule.

9. Emulsion cosmétique huile-dans-eau selon l'une des revendications précédentes, **caractérisée en ce qu'**elle contient comme émulsifiant non ionique ayant une valeur HLB dans la gamme de 3 à 6 le Stéareth-2 et en même temps comme émulsifiant non ionique ayant une valeur HLB dans la gamme de 12 à 18 le Stéareth-21.

10. Emulsion cosmétique huile-dans-eau selon l'une des revendications précédentes, **caractérisée en ce qu'**elle contient le Stéareth-2, le Stéareth-21 et l'éther de PPG-15-stéaryle.

11. Emulsion cosmétique huile-dans-eau selon la revendication 10, **caractérisée en ce qu'**elle contient au moins un polysaccharide, choisi parmi l'octénylsuccinate d'amidon d'aluminium et les phosphates de diamidon.

12. Emulsion cosmétique huile-dans-eau selon l'une des revendications précédentes, **caractérisée en ce qu'**elle contient au total au maximum 3% en poids, de préférence au maximum 1% en poids et de façon particulièrement préférée 0% en poids, à chaque fois sur la base du poids de toute l'émulsion, d'alcanols en C₁ à C₃ monovalents tels que l'éthanol ou l'isopropanol.

13. Utilisation cosmétique non thérapeutique d'une émulsion cosmétique huile-dans-eau selon l'une des revendications 1 à 12 dans le traitement anti-transpiration de la peau, notamment de la peau des aisselles et/ou de la peau du pied, avec une sensation de peau non grasse et/ou de séchage accéléré.

14. Procédé cosmétique, non thérapeutique de traitement anti-transpiration de la peau, notamment de la peau des aisselles et/ou de la peau du pied, **caractérisé en ce qu'**une émulsion huile-dans-eau selon l'une des revendications 1 à 12 est appliquée sur la peau dans une quantité efficace.
